# EUROPÄISCHE PATENTSCHRIFT

(11) **EP 3 515 900 B1**
(45) Veröffentlichungstag und Bekanntmachung des Hinweises auf die Patenterteilung: **11.08.2021**
(21) Anmeldenummer: 17761910.3
(22) Anmeldetag: 11.09.2017
(51) Int. Cl.: C07D 317/38

(54) **VERBINDUNGEN MIT ZWEI ODER MEHR EXOVINYLEN-CYCLOCARBONATEINHEITEN**
COMPOUNDS COMPRISING TWO OR MORE EXOVINYLENE CYCLIC-CARBONATE UNITS
COMPOSÉS COMPRENANT AU MOINS DEUX MOTIFS EXOVINYLÈNE-CYCLOCARBONATE

(30) Priorität: 21.09.2016 EP 16189808
(43) Veröffentlichungstag der Anmeldung: 31.07.2019
(73) Patentinhaber: BASF SE, 67056 Ludwigshafen am Rhein (DE)
(72) Erfinder: LICHT, Ulrike, 67056 Ludwigshafen (DE); LEONHARDT, Viktoria, 67063 Ludwigshafen (DE); MORMUL,Verena, 67056 Ludwigshafen (DE); SCHUMACHER, Karl-Heinz, 67056 Ludwigshafen (DE); BOERZSOENYI, Gabor, 76698 Ubstadt-Weiher (DE); KLOPSCH, Rainer, 67056 Ludwigshafen (DE)
(74) Vertreter: BASF IP Association
(86) Internationale Anmeldenummer: PCT/EP2017/072767
(87) Internationale Veröffentlichungsnummer: WO 2018/054713

(56) Entgegenhaltungen:
- WO-A1-2013/144299
- WO-A1-2015/010924
- WO-A1-2015/164692
- WO-A1-2016/202652

## Beschreibung

Die Erfindung betrifft Verbindungen mit zwei oder mehr Exovinylen-cyclocarbonateinheiten, wobei die Exovinylen-cyclocarbonateinheiten über mindestens eine organische, siloxanfreie Verbindungsgruppe miteinander verbunden sind, die nicht direkt mit den Exovinylen-Doppelbindungen verbunden ist und wobei durch Polymerisation von (Meth)acrylmonomeren gebildete Verbindungsgruppen ausgenommen sind. Beschrieben wird auch ein Verfahren zur Herstellung der Verbindungen, Zweikomponenten-Bindemittel, welche die Verbindungen enthalten und Verwendungen der Verbindungen.

Als Beschichtungsstoffe werden häufig zweikomponentige Systeme verwendet, in denen Isocyanatkomponenten mit Polyolkomponenten zu einem hochmolekularen Polyurethanpolymer reagieren. Diese Systeme werden entweder als lösemittelfreie und wasserfreie reaktive Einhundertprozentsysteme appliziert oder als in einem organischen Lösemittel gelöster Beschichtungsstoff. Die Beschichtungsstoffe werden mittels eines geeigneten Auftragssystems auf ein erstes Substrat aufgetragen und dann wird ggf. nach Verdampfen des Lösemittels ausgehärtet. Die in herkömmlichen Beschichtungsstoffen enthaltenen reaktiven Isocyanate stellen ein toxikologisches Risiko dar. Das betrifft zum einen die Verarbeitung dieser Beschichtungsstoffe bei deren Anwendung, weil die Isocyanate in der Regel eine hohe Toxizität und ein hohes allergenes Potential aufweisen. Zum anderen besteht die Gefahr, dass bei flexiblen Substraten nicht vollständig abreagiertes aromatisches Isocyanat durch das Substrat migriert und dort durch Wasseranteile zu carzinogenen aromatischen Aminen hydrolysiert. Gewünscht sind daher Isocyanat-freie Zweikomponentensysteme für härtbare Beschichtungszusammensetzungen mit guten Härtungseigenschaften möglichst bereits bei Raumtemperatur.

WO 2011/157671 offenbart eine cyclische Carbonatverbindung mit einer Doppelbindung direkt am Ringsystem, welche auch als Exovinylen-cyclocarbonat bezeichnet wird. Es werden keine Verbindungen mit zwei oder mehr Exovinylen-cyclocarbonatverbindungen beschrieben.

In der US 3,541,087 werden Verbindungen mit zwei Exovinylen-cyclocarbonatgruppen beschrieben welche durch eine direkte Bindung zwischen den Vinylengruppen miteinander verbunden sind. In der WO 2015/010924 werden Beschichtungsmassen beschrieben, enthaltend eine Verbindung mit mindestens zwei cyclischen Exovinylen-carbonatgruppen, welche über eine Siloxangruppe miteinander verbunden sind. In der WO 2013/144299 werden polymerisierbare Alkyliden-1,3-dioxolan-2-one beschrieben, wobei die Polymerisation über eine ethylenisch ungesättigte Gruppe erfolgt, die sich als Substituent über einen Spacer an der Alkylidengruppe befindet. In der WO 2015/039807 werden Beschichtungsmittelzusammensetzungen beschrieben, welche unter anderem eine oligomere oder polymere Verbindung mit mindestens zwei Alkyliden-1,3-dioxolan-2-on-Gruppen enthalten. Die Verknüpfung der Dioxolanone erfolgt dabei über die Alkylidengruppen.

In der WO 2015/164703 und in der WO 2015/164692 werden Polycyclocarbonatverbindungen und daraus hergestellte Polymere beschrieben. Ausführbare Offenbarungen sind nur für Verbindungen mit zwei Polycyclocarbonatgruppen beschrieben.

Exovinylen-cyclocarbonate können bei Raumtemperatur mit Aminen zu Urethanen reagieren und sind damit eine potentielle Alternative zur Polyurethanbildung durch Isocyanat/Alkohol-Reaktionen. In der unveröffentlichten europäischen Patentanmeldung mit der Anmeldenummer 15164849.0 werden Acrylatmonomere beschrieben, welche eine Exovinylen-cyclocarbonatgruppe enthalten. Multifunktionelle Derivate (Copolymere) aus diesen Monomeren werden in der unveröffentlichten europäischen Patentanmeldung mit der Anmeldenummer 15172703.9 beschrieben. Allerdings müssen diese Derivate und Monomere über mehrere Stufen mit deutlichem Ausbeuteverlust synthetisiert werden.

Die Aufgabe der Erfindung bestand darin, aus möglichst einfachen Vorstufen eine Verbindung zu synthetisieren, die mehr als eine exo-Vinylencyclocarbonatgruppe enthält und als Reaktivbaustein für isocyanatfreie 2-Komponeten Beschichtungsmittel dienen kann, die möglichst bereits bei Raumtemperatur aushärten können.

Gegenstand der Erfindung ist eine Verbindung mit zwei oder mehr Exovinylen-cyclocarbonateinheiten, wobei die Exovinylen-cyclocarbonateinheiten über mindestens eine organische, siloxanfreie Verbindungsgruppe miteinander verbunden sind, wobei sich die Verbindungsgruppe nicht zwischen den Exovinylengruppen befindet und wobei durch Polymerisation von (Meth)acrylmonomeren gebildete Verbindungsgruppen ausgenommen sind. Die Verbindungsgruppe weist für den Fall, dass die erfindungsgemäße Verbindung zwei Exovinylen-cyclocarbonateinheiten aufweist, mindestens eine Acetalgruppe auf. Die Verbindungsgruppe weist mindestens eine Acetalgruppe auf und es handelt sich um Verbindungen der nachstehend beschriebenen Formel (Vb), (Vc, (IVb) oder (VI).

Es handelt sich bei den Exovinylen-cyclocarbonateinheiten um 5-Vinyliden-1,3-dioxolan-2-on-Einheiten der allgemeinen Formel (I) 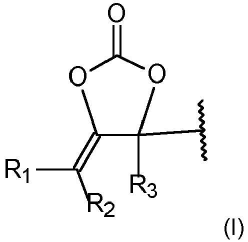 wobei die mindestens eine organische, siloxanfreie Verbindungsgruppe sich zwischen den 4-Positionen der 5-Vinyliden-1,3-dioxolan-2-on-Einheiten befindet und wobei R1 und R2 für Wasserstoff stehen und R3 für eine Methylgruppe steht.

Geeignete erfindungsgemäße Verbindungen sind solche der Formel (Vb) 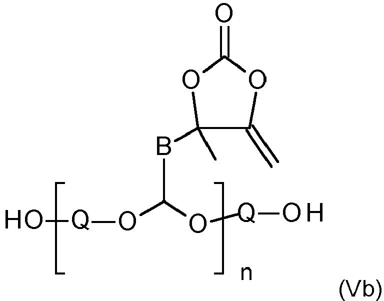
wobei B eine Alkylengruppe mit vorzugsweise 1 bis 6 C-Atomen, z.B. Butylen, Propylen, Ethylen oder besonders bevorzugt Methylen bedeutet;
Q eine von einem Poylol abgeleitete Gruppe bedeutet, wobei ein Polyol ein Alkohol mit mindestens zwei Hydroxygruppen ist;
und n eine Zahl größer oder gleich 2 ist, vorzugsweise 2 bis 10 oder 3 bis 10.

Als von einem Poylol abgeleitete Gruppen sind insbesondere die von den unten genannten alkoholischen Härtern abgeleiteten Gruppen geeignet, vorzugsweise ausgewählt aus Ethylenglykol, 1,3-Propandiol, 1,4-Butandiol, Diethylenglykol, Triethylenglykol, Neopentylglykol, 1,5-Pentandiol, 1,6-Hexandiol, 1,8-Octandiol, 3-Methyl-1,5-pentandiol, 1,4-bis-Hydroxymethyl-cyclohexan, 1,6-bis-Hydroxymethyl-cyclohexan, Glycerin, Diglycerin, Polyethylenglykol, Polypropylenglykol, Poly-THF, Pentaerythrit, Dipentaerythrit, Zuckeralkohole wie Sorbit und Mannit.

Erfindungsgemäße Verbindungen sind auch solche der Formel (Vc) 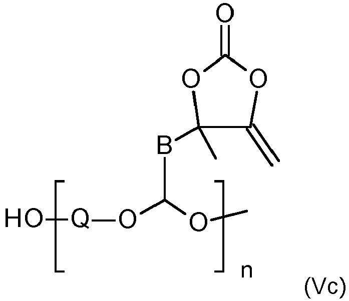 wobei B, Q und n die gleiche Bedeutung haben wie bei Formel (Vb).

Erfindungsgemäße Verbindungen sind auch solche der Formel (IVb) 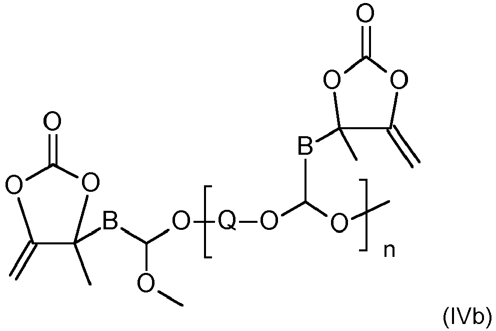
wobei B und Q die gleiche Bedeutung haben wie bei Formel (Vb) und
n eine Zahl größer oder gleich 1 ist, vorzugsweise 1 bis 10 oder 2 bis 10.

Erfindungsgemäße Verbindungen sind auch solche der Formel (VI) 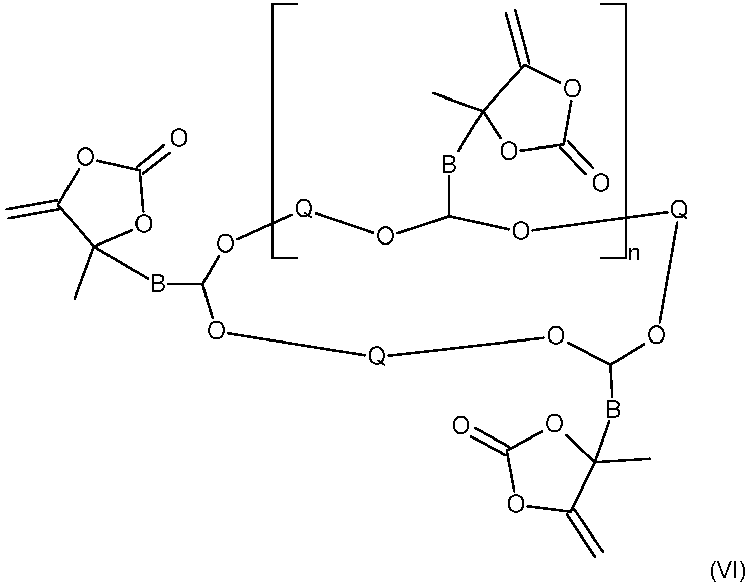
wobei B und Q die gleiche Bedeutung haben wie bei Formel (Vb) und
n eine Zahl größer oder gleich 0 ist, vorzugsweise 0 bis 10 oder 1 bis 5.

Als Beispiele für erfindungsgemäße Verbindungen seien die folgenden bevorzugten Verbindungen genannt:
Verbindung der Formel (VIa): 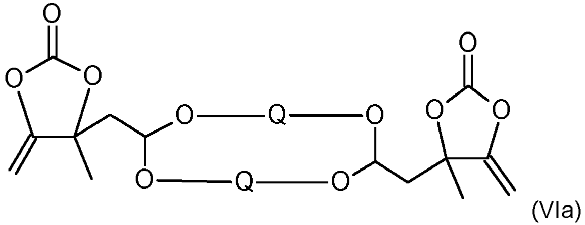
Verbindung der Formel (IVc): 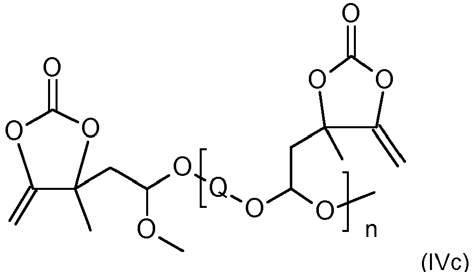 mit n = 1, 2 oder 3.
Verbindung der Formel (Vd): 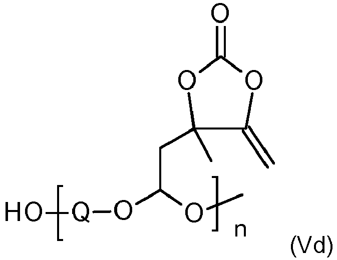 mit n größer oder gleich 2, vorzugsweise 2 oder 3.
Verbindung der Formel (Ve): 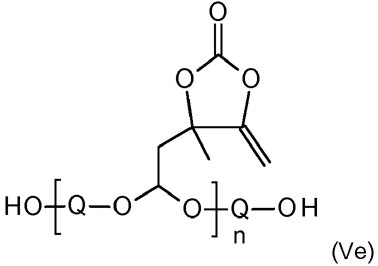 mit n größer oder gleich 2, vorzugsweise 2 oder 3.

Wobei -O-Q-O- in den Formeln VIa, IVc, Vd und Ve jeweils für den Rest eines zweiwertigen Alkohols steht, vorzugsweise ausgewählt aus Ethylenglykol, 1,3-Propandiol, 1,4-Butandiol, Diethylenglykol, Triethylenglykol, Neopentylglykol, 1,5-Pentandiol, 1,6-Hexandiol, 1,8-Octandiol, 3-Methyl-1,5-pentandiol, 1,4-bis-Hydroxymethyl-cyclohexan, 1,6-bis-Hydroxymethyl-cyclohexan, Glycerin, Diglycerin, Polyethylenglykol, Polypropylenglykol, Poly-THF, Pentaerythrit, Dipentaerythrit, Zuckeralkohole wie Sorbit und Mannit.

Erfindungsgemäße Verbindungen können beispielsweise hergestellt werden durch Umacetalisierung eines Exovinylen-cyclocarbonats der Formel (VII) 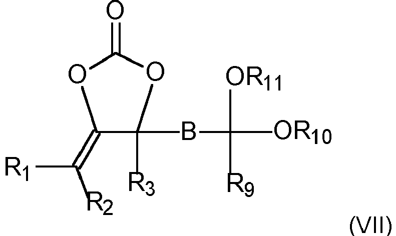 wobei R10 und R11 unabhängig voneinander einen organischen Rest bedeuten. R1 bis R3 haben die gleiche Bedeutung wie bei Formel (I). R9 steht für ein H-Atom. R10 und R11 stehen vorzugsweise für eine C1- bis C10-Alkylgruppe, eine C1- bis C6-Alkylgruppe oder eine C1- bis C3-Alkylgruppe, besonders bevorzugt für eine Methylgruppe. Vorzugsweise stehen bei Verbindungen der Formel (VII) R¹⁰ und R¹¹ unabhängig voneinander für Kohlenwasserstoffgruppen, insbesondere für C₁- bis C₁₀-Alkylgruppen oder Alkylgruppen mit 1 bis 4 C-Atomen, besonders bevorzugt für Methylgruppen. B steht für eine Alkylengruppe mit 1 bis 4 C-Atomen, insbesondere für Methylen.

Besonders bevorzugt ist eine Verbindung der Formel (Vlla):

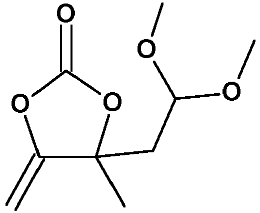

Für eine Umacetalisierung geeignete Exovinylen-cyclocarbonate können beispielsweise hergestellt werden durch ein Verfahren, bei dem
- in einer ersten Stufe eine Verbindung mit einer endständigen Dreifachbindung mit einem Alkanon oder Alkanal, welches eine Acetalgruppe enthält, umgesetzt wird, wobei sich die Dreifachbindung an die Carbonylgruppe des Alkanon oder Alkanals unter Ausbildung einer Hydroxyverbindung addiert,
- in einer zweiten Stufe mit Kohlendioxid der Ringschluss zur Carbonatgruppe erfolgt.

### Zu Stufe 1

Die Umsetzung der ersten Stufe ist eine an sich bekannte Addition von Dreifachbindungen an eine Carbonylgruppe. Geeignete Verbindungen mit einer endständigen Dreifachbindung sind insbesondere Verbindungen der Formel (VIII)

Y-CH≡CH,

wobei Y für ein H-Atom, eine Kohlenwasserstoffgruppe mit 1 bis 10 C- Atomen, z.B. eine Alkyl- oder Arylgruppe oder eine Schutzgruppe mit maximal 10 C-Atomen steht. Soweit es sich bei Y nicht um eine Schutzgruppe handelt, bestimmen die Substituenten des Y-substituierten C-Atoms die späteren Reste R1, R2, R5 und R6 in den Formeln (I) bis (V). Ausgehend von Formel (VIII) ist daher einer der Reste R1 oder R2 bzw. einer der Reste R5 oder R6 in Formeln (I) bis (V) ein H-Atom und der jeweils andere Y. Die bevorzugten Bedeutungen von Y entsprechen daher den obigen bevorzugten Bedeutungen von R1, R2, R5 und R6.

Y kann jedoch auch für eine Schutzgruppe stehen. Schutzgruppen werden während oder nach der erfolgten Synthese wieder abgespalten, so dass in diesem Fall die späteren Reste R1 und R2 bzw. R5 und R6 in Formeln (I) bis (V) und (VII) jeweils für ein H-Atom stehen. Eine geeignete Schutzgruppe ist z.B. die Trimethylsilylgruppe (kurz TMS)

Zur Durchführung der Additionsreaktion sind verschiedene Methoden bekannt. Vorzugsweise werden die Ausgangsverbindungen in Gegenwart einer starken Base umgesetzt. Bevorzugte starke Basen sind Metallalkoholate. Es handelt sich dabei vorzugsweise um Metallsalze aliphatischer Alkohole, insbesondere Metallsalze von C1- bis C8-, vorzugsweise C2- bis C6- Alkoholen, wie Ethanol, n-Propanol, iso-Propanol, n-Butanol oder tertiär-Butanol. Bei den Metallkationen der Metallalkoholate handelt es sich vorzugsweise um Alkali-kationen, z.B. die Kationen von Natrium oder Kalium. Als bevorzugtes Metallalkoholat seien z.B. Kalium-tert.-butylat, Natrium-tert.-butylat, Kaliumisopropylat und Natriumisopropylat genannt.

Die Umsetzung wird vorzugsweise in Gegenwart eines Lösemittels durchgeführt. Als Lösemittel bevorzugt sind inerte Lösemittel; diese enthalten keine reaktiven Gruppen, welche mit den Ausgangsverbindungen reagieren. Besonders bevorzugt sind inerte, polare, aprotische Lösemittel. Als solche genannt seien z.B. cyclische Etherverbindungen, insbesondere THF. Die Umsetzung ist im Allgemeinen exotherm, daher wird bei der Umsetzung vorzugsweise gekühlt. Die Temperatur des Reaktionsgemisches beträgt vorzugsweise maximal 50 °C, insbesondere maximal 25°C; vorzugsweise liegt sie zwischen 0 und 25°C.

Zur Aufarbeitung des erhaltenen Produktgemisches kann Wasser, gegebenenfalls Säure und gegebenenfalls ein unpolares organisches Lösemittel zugegeben. Soweit das Wertprodukt der 1.Stufe bereits eine eigenständige organische Phase ausbildet, kann auf das organische Lösemittel verzichtet werden. Es bilden sich zwei Phasen aus, von denen die organische Phase das Produkt der 1. Stufe (Additionsprodukte) enthält. Die organische Phase kann zur Entfernung von Wasser getrocknet werden. Lösemittel kann leicht destillativ abgetrennt werden. Das Produkt kann durch Vakuumdestillation in Reinform erhalten werden. Alternativ kann die Aufarbeitung auch nach üblichen Verfahren der Kristallisation oder Extraktion erfolgen, insbesondere wenn das Produkt der 1. Stufe einen sehr hohen Siedepunkt hat.

Die verwendeten Alkanone oder Alkanale enthalten eine Acetalgruppe. Bevorzugte Alkanone oder Alkanale mit einer Acetalgruppe sind solche der Formel IX 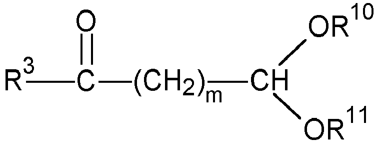 worin R³ für ein H-Atom oder eine C1- bis C10 Alkylgruppe steht, m für 0 oder eine ganze Zahl von 1 bis 10 steht. m steht für 0 oder eine ganze Zahl von 1 bis 10. Vorzugsweise steht m für eine ganze Zahl von 1 bis 10, insbesondere für eine ganze Zahl von 1 bis 6, ganz besonders bevorzugt steht m für 1. Die Gruppe 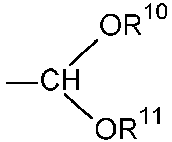 steht für die Acetalgruppe, wobei R¹⁰ und R¹¹ für Kohlenwasserstoffgruppen stehen, insbesondere für C₁- bis C₁₀-Alkylgruppen oder Alkylgruppen mit 1 bis 4 C-Atomen, besonders bevorzugt Methylgruppen. R³ entspricht dem R³ bzw. R⁴ in den Formeln I bis V und VII und hat die entsprechenden Bedeutungen und bevorzugten Bedeutungen; in einer bevorzugten Ausführungsform ist R³ eine Methylgruppe.

Zur Durchführung der Additionsreaktion sind verschiedene Methoden bekannt. Vorzugsweise werden die Ausgangsverbindungen in Gegenwart einer starken Base umgesetzt. Bevorzugte starke Basen sind Metallalkoholate. Es handelt sich dabei vorzugsweise um Metallsalze aliphatischer Alkohole, insbesondere Metallsalze von C1- bis C8-, vorzugsweise C2- bis C6- Alkoholen, wie Ethanol, n-Propanol, iso-Propanol, n-Butanol oder tertiär-Butanol. Bei den Metallkationen der Metallalkoholate handelt es sich vorzugsweise um Alkalikationen, z.B. die Kationen von Natrium oder Kalium. Als bevorzugtes Metallalkoholat seien z.B. Kalium-tert.-butylat, Natrium-tert.-butylat, Kaliumisopropylat und Natriumisopropylat genannt.

Die Umsetzung wird vorzugsweise in Gegenwart eines Lösemittels durchgeführt. Als Lösemittel bevorzugt sind inerte Lösemittel; diese enthalten keine reaktiven Gruppen, welche mit den Ausgangsverbindungen reagieren. Besonders bevorzugt sind inerte, polare, aprotische Lösemittel. Als solche genannt seien z.B. cyclische Etherverbindungen, insbesondere THF. Die Umsetzung ist im Allgemeinen exotherm, daher wird bei der Umsetzung vorzugsweise gekühlt. Die Temperatur des Reaktionsgemisches beträgt vorzugsweise maximal 50 °C, insbesondere maximal 25°C; vorzugsweise liegt sie zwischen 0 und 25°C.

Zur Aufarbeitung des erhaltenen Produktgemisches kann Wasser, gegebenenfalls Säure und gegebenenfalls ein unpolares organisches Lösemittel zugegeben. Soweit das Wertprodukt der 1.Stufe bereits eine eigenständige organische Phase ausbildet, kann auf das organische Lösemittel verzichtet werden. Es bilden sich zwei Phasen aus, von denen die organische Phase das Produkt der 1. Stufe (Additionsprodukte) enthält. Die organische Phase kann zur Entfernung von Wasser getrocknet werden. Lösemittel kann leicht destillativ abgetrennt werden. Das Produkt kann durch Vakuumdestillation in Reinform erhalten werden. Alternativ kann die Aufarbeitung auch nach üblichen Verfahren der Kristallisation oder Extraktion erfolgen, insbesondere wenn das Produkt der 1.Stufe einen sehr hohen Siedepunkt hat.

### Zu Stufe 2

In Stufe 2 erfolgt der Ringschluss mit Kohlendioxid zur Ausbildung der cyclischen Carbonatgruppe. Produkt der zweiten Stufe ist eine Verbindung mit einer cyclischen Carbonatgruppe und einer Acetalgruppe. Dazu wird Kohlendioxid vorzugsweise gasförmig oder in überkritischem Zustand unter Druck mit der Ausgangsverbindung in Kontakt gebracht. Die Umsetzung wird daher vorzugsweise in einem Autoklaven durchgeführt. Kohlendioxid kann auch in Gemisch mit Inertgas verwendet werden.

Die Umsetzung erfolgt vorzugsweise in Gegenwart von Katalysatoren. Vorzugsweise erfolgt sie in Gegenwart einer Base als Katalysator oder besonders bevorzugt in Gegenwart eines Katalysatorsystems aus einer Base und einem Metallsalz. Als Base sind Verbindungen mit mindestens einer tertiären Aminogruppe, z.B. mit ein bis drei tertiären Aminogruppen bevorzugt. Derartige Basen sind bekannt. Sie haben üblicherweise ein Molgewicht unter 500 g/mol, insbesondere unter 300 g/mol. Insbesondere handelt es sich dabei um aliphatische oder cycloaliphatische Verbindungen.

Als Basen genannt seien z.B.
TMTACN (N,N',N"-trimethyl-1,4,7-triazacyclononan),
PMDETA (Pentamethyldiethylentriamin)
TMEDA (Tetramethylethylendiamin)
DBU (1,8-Diazabicyclo[5.4.0]undec-7-en) oder
DBN (1,5-Diazabicyclo[4.3.0]non-5-en).

Bei dem Metallsalz handelt es sich vorzugsweise um Salze mit ein bis drei wertigen Kationen, insbesondere Kationen des Cu, Ag oder Au. Das Anion der Metallsalze ist vorzugsweise ein Carboxylat, insbesondere ein C1- bis C6-Carboxylat. Als bevorzugte Metallsalze genannt seien Silberacetat oder Kupferacetat.

Als Katalysatoren kommen auch Phosphane in Betracht. Insbesondere handelt es sich dabei um Trialkyl- oder Triarylphosphane. Diese können allein oder ebenfalls in Kombination mit einem Metallsalz eingesetzt werden.

Die Umsetzung wird vorzugsweise bei einem Druck von 1 bis 100 bar, insbesondere 5 bis 70 bar durchgeführt. Die Temperatur des Reaktionsgemisches beträgt vorzugsweise 10 bis 100°C, insbesondere 10 bis 80°C. Die Umsetzung kann z.B. durch Gaschromatographie überwacht werden.

Nach Abkühlung und Druckentspannung kann das erhaltene Produkt aufgearbeitet werden. Es kann ein organisches Lösemittel, vorzugsweise ein inertes, hydrophobes organisches Lösemittel wie Dichlormethan oder Toluol, und wässrige Säure, z.B. wässrige HCl, zugegeben werden, so dass sich zwei Phasen bilden. Die organische Phase enthält das gewünschte Produkt. Aus der organischen Phase kann Wasser durch Trocknung entfernt werden. Lösemittel kann destillativ entfernt werden. Das Produkt kann durch Destillation gereinigt werden. Eine schonende und daher bevorzugte Destillation ist z.B. die Destillation in einem Dünnschichtverdampfer. Insbesondere sind dazu Dünnschichtverdampfer mit Wischersystem geeignet. Alternativ kommt bei hohen Siedepunkten des Produkts der zweiten Stufe auch eine Aufarbeitung und Reinigung durch Kristallisation oder Extraktion in Betracht.

### Umacetalisierung:

Die Umacetalisierung, z.B. von Reaktionsprodukten der Stufe 2, kann mit Alkoholen unter Katalyse mit Lewissäuren oder Protonensäuren durchgeführt werden, wobei die letzteren bessere Ausbeuten ergeben. Saure Katalysatoren sind z.B. saure anorganische, metallorganische oder organische Katalysatoren oder Gemische aus mehreren sauren anorganischen, metallorganischen oder organischen Katalysatoren. Als saure anorganische Katalysatoren sind beispielsweise Schwefelsäure, Sulfate und Hydrogensyulfate, wie Natriumhydrogensulfat, Phosphorsäure, Phosphonsäure, hypophosphorige Säure, Aluminiumsulfathydrat, Alaun, saures Kieselgel (pH ≤ 6, insbesondere ≤ 5) und saures Aluminiumoxid zu nennen. Weiterhin sind beispielsweise Alumiumverbindungen der allgemeinen Formel Al(OR¹)₃ und Titanate der allgemeinen Formel Ti(OR¹)₄ als saure anorganische Katalysatoren einsetzbar, wobei die Reste R¹ jeweils gleich oder verschieden sein können und unabhängig voneinander gewählt sind aus C₁-C₂₀-Alkylresten, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, isoHexyl, sec.-Hexyl, n-Heptyl, iso-Heptyl, n-Octyl, 2-Ethylhexyl, n-Nonyl, n-Decyl, n-Dodecyl, n-Hexadecyl oder n-Octadecyl; C₃-C₁₂-Cycloalkylresten, beispielsweise Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl. Bevorzugte saure metallorganische Katalysatoren sind beispielsweise gewählt aus Dialkylzinnoxiden R¹₂SnO oder Dialkylzinnestern R¹₂Sn(OR²)₂ wobei R¹ wie oben stehend definiert ist und gleich oder verschieden sein kann. R² kann die gleichen Bedeutungen haben wie R¹ und zusätzlich C₆-C₁₂-Aryl sein, beispielsweise Phenyl, o-, m- oder p-Tolyl, Xylyl oder Naphthyl. R² kann jeweils gleich oder verschieden sein. Beispiele sind für zinnorganische Katalysatoren sind Zinn(II)-n-octanoat, Zinn-(II)-2-ethylhexanoat, Zinn-(II)-laurat, Dibutylzinnoxid, Diphenylzinnoxid, Dibutylzinndichlorid, Dibutylzinndiacetat, Dibutylzinndilaurat, Dibutylzinndimaleat oder Dioctylzinndiacetat. Besonders bevorzugte Vertreter für saure metallorganische Katalysatoren sind Dibutylzinnoxid, Diphenylzinnoxid und Dibutylzinndilaurat.

Bevorzugte saure organische Katalysatoren sind saure organische Verbindungen mit beispielsweise Phosphatgruppen, Sulfonsäuregruppen, Sulfatgruppen oder Phosphonsäuregruppen. Man kann auch saure lonentauscher als saure organische Katalysatoren einsetzen, beispielsweise Sulfonsäuregruppen-haltige Polystyrolharze, die mit etwa 2 mol-% Divinylbenzol vernetzt sind. Besonders bevorzugt sind Sulfonsäuren wie beispielsweise p-Toluolsulfonsäure oder Methansulfonsäure; sowie Trifluoressigsäure. Die Katalysatoren können homogen oder auch heterogen oder geträgert sein. Stärkere Säuren tendieren zu Verfärbungen der Reaktionsprodukte, schwächere Säuren benötigen höhere Temperaturen.

Die Alkohole können primär sekundär oder tertiär sein, wobei primäre Alkohole bevorzugt sind. Diole sind besonders bevorzugt, da sie zu linearen Oligomeren führen. Setzt man Polyole mit drei oder mehr Hydroxygruppen ein, können zur Regelung des Molekulargewichtes und der Verzweigung auch Monoole zusätzlich mitverwendet werden. Mischungen von Alkoholen sind ebenfalls möglich. Die Molekulargewichte der Diole betragen vorzugsweise von 62 bis 5000 g/mol, besonders bevorzugt von 90 bis 2000 g/mol (im Fall von Polymeren: zahlenmittleres Molekulargewicht Mn aus Endgruppenanalyse).

Das Verhältnis von OR-Gruppen der Acetalgruppen zu OH Gruppen kann 4:1 bis 1:4 sein, bevorzugt 2:1 bis 1:2, ganz besonders bevorzugt 1,5:1 bis 1:1,5.

Die Reaktion kann in verschiedenen Lösungsmitteln durchgeführt werden, bevorzugt sind polare Lösungsmittel wie z.B. Acetonitril. Reaktionstemperaturen liegen zwischen Raumtemperatur (20°C) und 100°C, bevorzugt < 80°C, besonders bevorzugt < 60°C. Besonders bevorzugt wird die Reaktion unter Vakuum durchgeführt zur effektiven Entfernung des Methanols aus dem Gleichgewicht. Dazu ist es besonders bevorzugt, wenn die Reaktion über weite Strecken lösungsmittelfrei geführt wird. So kann die Reaktionsmischung zur besseren Homogenisierung erst in Lösung angesetzt werden und das Lösungsmittel dann unter Vakuum entfernt werden. Es kann auch zurückgewonnen und wiederverwendet werden.

Die Aufarbeitung der erfindungsmäßen Reaktionsprodukte (oligomere Exovinylen-cyclocarbonatacetale) kann durch einfaches Auswaschen des Katalysators erfolgen. Ist er geträgert, muss nur filtriert werden. Höhere Reinheiten ergeben sich durch Ausschütteln mit Wasser, Puffern oder ganz schwachen Basen und Trocknung mit Trockenmitteln. Auch Fällungen in Wasser und Nichtlösemitteln sind möglich, wobei darauf zu achten ist, dass die Acetalgruppen nicht hydrolysiert werden.

Die erfindungsgemäßen Exovinylen-cyclocarbonate haben gewichtsmittlere Molekulargewichte Mw vorzugsweise zwischen 500 und 50 0000 g/mol, besonders bevorzugt zwischen 1000 und 10 000 g/mol, gemessen über Gelpermeationschromatographie (GPC in THF; Standard Polystyrol). Die Viskosität beträgt vorzugsweise 0,5 bis 5000 Pa s (gemessen als Nullviskosität). Die Nullviskosität ist der Grenzwert der Viskositätsfunktion bei unendlich niedrigen Scherraten. Sie wird gemessen mit einem Anton Paar Rheometer MCR 100 (US 200 Auswertesoftware) in Platte/Platte Geometrie. Die Proben werden in oszillatorischer Scherung bei kleiner Scheramplitude von 10% und bei einer Temperatur von 23 °C vermessen, Kreisfrequenzrampe log 100-0,1 1/s, Messspalt 0,5 mm, Auswertung nach Carreau-Gahleitner I, Stempeldurchmesser 25 mm.

Die erfindungsgemäßen Exovinylen-cyclocarbonate können ohne Zerstörung des Exovinylencarbonat-Rings mit Isocyanaten, Säurechloriden und/oder Säureanhydriden kettenverlängert werden. Sie können auch unter Ringöffnung mit Polyaminen zu Polyurethanen umgesetzt werden. Sie können auch unter Ringöffnung mit weiteren Polyolen und stark basischer Katalyse zu Polycarbonaten umgesetzt werden.

Gegenstand der Erfindung ist auch ein Verfahren zur Herstellung einer Verbindung mit zwei oder mehr Exovinylen-cyclocarbonateinheiten, wobei eine Verbindung, welche eine Exovinylen-cyclocarbonateinheit und mindestens eine Acetalgruppe aufweist, umgesetzt wird in einer Umacetalisierungsreaktion mit mindestens einer Verbindung ausgewählt aus der Gruppe bestehend aus Diolen und Polyolen. Die Umsetzung wird vorzugsweise unter Katalyse mit Lewissäuren oder mit Protonensäuren durchgeführt.

Gegenstand der Erfindung sind auch die Reaktionsprodukte des Verfahrens.

Gegenstand der Erfindung ist auch die Verwendung der erfindungsgemäßen Verbindungen als Bestandteil von Beschichtungsstoffen, Lacken, Farben, Tinten, Baustoffen, Elastomeren, Schäumen oder zur Bindung von Fasern und/oder Partikeln.

Abmischungen der erfindungsgemäßen Verbindungen mit einer mehrfunktionellen Härterkomponente können als Zwei-Komponenten Bindemittel eingesetzt werden. Gegenstand der Erfindung sind daher auch Verwendungen von Zwei-Komponenten Bindemitteln enthaltend in einer ersten Komponente mindestens eine erfindungsgemäße Verbindung und in einer zweiten Komponente mindestens einen mehrfunktionellen Härter, welcher mindestens zwei funktionelle Gruppen aufweist ausgewählt aus der Gruppe bestehend aus primären Aminogruppen, sekundären Aminogruppen, Hydroxygruppen, Phosphingruppen, Phosphonatgruppen und Mercaptangruppen. Vorzugsweise enthält das Zwei-Komponenten Bindemittel mindestens einen Katalysator für die Katalysierung der Reaktion der Exovinylen-cyclocarbonatgruppen mit den funktionellen Gruppen des Härters. Das Zwei-Komponenten Bindemittel kann als Lösung in einem organischen Lösemittel oder als lösemittel- und wasserfreies Einhundertprozentsystem appliziert werden.

Vorzugsweise sind die funktionellen Gruppen des Härters ausgewählt aus aliphatischen Hydroxylgruppen, aliphatischen primären Aminogruppen, aliphatischen sekundären Aminogruppen, aliphatischen Phosphingruppen, aliphatischen Phosphonatgruppen und aliphatischen Mercaptangruppen.

Unter Zweikomponenten-Bindemitteln (2K-Bindemittel) versteht man ein Bindemittel, das wenigstens zwei polyfunktionelle Bindemittelbestandteile enthält, die miteinander unter Bindungsbildung reagieren und dabei ein polymeres Netzwerk ausbilden. Die erfindungsgemäßen Polymere können aufgrund der darin vorhandenen Alkyliden-1,3-dioxolan-2-on-Gruppen mit zahlreichen nucleophilen Gruppen unter Bindungsbildung reagieren. Beispiele für solche nucleophilen Gruppen sind vor allem aliphatische Hydroxylgruppen, aliphatische primäre und sekundäre Aminogruppen, Phosphingruppen, insbesondere aliphatische Phosphingruppen, Phosphonatgruppen, insbesondere aliphatische Phosphonatgruppen, sowie analoge Phosphorverbindungen, außerdem Mercaptangruppen, insbesondere aliphatische Mercaptangruppen.

Dementsprechend enthalten 2K-Bindemittelzusammensetzungen neben wenigstens einem erfindungsgemäßen Polymer vorzugsweise zusätzlich wenigstens eine Verbindung, die wenigstens 2 funktionelle Gruppen F, z.B. 2, 3, 4, 5, 6, 7, 8, 9 oder 10 funktionelle Gruppen F, aufweist, welche unter aliphatischen Hydroxylgruppen, aliphatischen primären oder sekundären Aminogruppen, aliphatischen Phosphingruppen, aliphatischen Phosphonatgruppen u.ä. Gruppen und aliphatischen Mercaptangruppen ausgewählt sind. Diese Verbindungen werden im Folgenden auch als Härter bezeichnet. Bevorzugte funktionelle Gruppen F sind aliphatische Hydroxylgruppen und aliphatische primäre und sekundäre Aminogruppen. Vorzugsweise wird die Menge an Härter so gewählt, dass das Molverhältnis von funktionellen Alkyliden-1,3-dioxolan-2-on-Gruppen der Formel I zu den funktionellen Gruppen F im Härter im Bereich von 1 : 10 bis 10:1, insbesondere im Bereich von 5 : 1 bis 1 : 5 und speziell im Bereich von 1 : 2 bis 2 : 1 liegt.

Der Härter kann eine niedermolekulare Substanz sein, d. h., sein Molekulargewicht liegt unterhalb 500 g/mol, oder eine oligomere oder polymere Substanz, die ein zahlenmittleres Molekulargewicht oberhalb 500 g/mol aufweist.

Zu den erfindungsgemäß bevorzugten Härtern gehören aminische Härter, d.h. Härter, die wenigstens zwei primäre oder sekundäre Aminogruppen aufweisen, und alkoholische Härter, also Verbindungen, die wenigstens zwei Hydroxylgruppen aufweisen.

Zu den aminischen Härtern, im Folgenden auch Aminhärter, zählen beispielsweise aliphatische und cycloaliphatische Polyamine, aromatische und araliphatische Polyamine sowie polymere Amine, z. B. Aminoplaste und Polyamidoamine. Aminhärter vernetzen Polymere mit 1,3-Dioxolan-2-on-Gruppen, im Folgenden auch Carbonatpolymere genannt, durch Reaktion der primären oder sekundären Aminofunktionen der Polyamine mit den 1,3-Dioxolan-2-on-Gruppen der Carbonatpolymere unter Ausbildung von Urethanfunktionen. Bevorzugte Polyaminhärter weisen im Mittel wenigstens zwei primäre oder sekundäre Aminogruppen pro Molekül, z. B. zwei, drei oder vier primäre oder sekundäre Aminogruppen pro Molekül, auf. Sie können auch zusätzlich ein oder mehrere tertiäre Aminogruppen enthalten. Geeignete Polyamine sind beispielsweise
- aliphatische Polyamine wie Ethylendiamin, 1,2- und 1,3-Propandiamin, Neopentandiamin, Hexamethylendiamin, Octamethylendiamin, 1,10-Diaminodecan, 1,12-Diaminododecan, Diethylentriamin, Triethylentetramin, Tetraethylenpentamin, 2,2-Dimethylpropylendiamin, Trimethylhexamethylendiamin, 1-(3-Aminopropyl)-3-aminopropan, 1,3-Bis-(3-aminopropyl)propan, 4-Ethyl-4-methylamino-1-octylamin und dergleichen;
- cycloaliphatische Diamine wie 1,2-Diaminocyclohexan, 1,2-, 1,3-, 1,4-Bis(aminomethyl)-cyclohexan, 1-Methyl-2,4-diaminocyclohexan, N-Cyclohexylpropylen-1,3-diamin, 4-(2-Aminopropan-2-yl)-1-methylcyclohexan-1-amin, Isophorondiamin, 4,4'-Diaminodicyclohexylmethan (Dicykan), 3,3'-Dimethyl-4,4'-diaminodicyclohexylmethan, 3,3',5,5'-Tetramethyl-4,4'-diaminodicyclohexylmethan, 4,8-Diamino-tricyclo[5.2.1.0]decan, Norbornandiamin, Menthandiamin, Menthendiamin und dergleichen;
- aromatische Diamine wie Toluylendiamin, Xylylendiamin, insbesondere meta-Xylylendiamin (MXDA), Bis(4-aminophenyl)methan (MDA oder Methylendianilin), Bis(4-aminophenyl)sulfon (auch als DADS, DDS oder Dapson bekannt) und dergleichen;
- cyclische Polyamine wie Piperazin, N-Aminoethylpiperazin und dergleichen;
- Polyetheramine, insbesondere difunktionelle und trifunktionelle primäre Polyetheramine auf der Basis von Polypropylenglykol, Polyethylenglykol, Polybutylenoxid, Poly-(1,4-butandiol), Poly-Tetrahydrofuran (Poly-THF) oder Polypentylenoxid, z. B. 4,7,10-Trioxatridecan-1,3-diamin, 4,7,10-Trioxatridecan-1,13-diamin, 1,8-Diamino-3,6-dioxaoctan (XTJ-504, Fa. Huntsman), 1,10-Diamino-4,7-dioxadecan (XTJ-590, Fa. Huntsman), 1,12-Diamino-4,9-dioxadodecan (Fa. BASF SE), 1,3-Diamino-4,7,10-trioxatridecan (Fa. BASF SE), primäre Polyetheramine auf der Basis von Polypropylenglykol mit einer mittleren Molmasse von 230 wie z. B. Polyetheramine D 230 (Fa. BASF SE) oder Jeffamine® D 230 (Fa. Huntsman), difunktionelle, primäre Polyetheramine auf der Basis von Polypropylenglykol mit einer mittleren Molmasse von 400, z. B. Polyetheramine D 400 (Fa. BASF SE) oder Jeffamine® XTJ 582 (Fa. Huntsman), difunktionelle, primäre Polyetheramine auf Basis von Polypropylenglykol mit einer mittleren Molmasse von 2000 wie z. B. Polyetheramine D 2000 (Fa. BASF SE), Jeffamine® D2000 oder Jeffamine® XTJ 578 (jeweils Fa. Huntsman), difunktionelle, primäre Polyetheramine auf der Basis von Propylenoxid mit einer mittleren Molmasse von 4000 wie z. B. Polyetheramin D 4000 (Fa. BASF SE), trifunktionelle, primäre Polyetheramine hergestellt durch Reaktion von Propylenoxid mit Trimethylolpropan, gefolgt durch eine Aminierung der endständigen OH-Gruppen mit einer mittleren Molmasse von 403 wie z. B. Polyetheramine T 403 (Fa. BASF SE) oder Jeffamine® T 403 (Fa. Huntsman), trifunktionelle, primären Polyetheramin, hergestellt durch Reaktion von Propylenoxid mit Glycerin, gefolgt durch eine Aminierung der endständigen OH-Gruppen mit einer mittleren Molmasse von 5000 wie z. B. Polyetheramine T 5000 (Fa. BASF SE) oder Jeffamine® T 5000 (Fa. Huntsman), aliphatische Polyetheramine, die aus einem mit Propylenoxid gepfropften Polyethylenglykol aufgebaut sind und eine mittlere Molmasse von 600 aufweisen, wie z. B. Jeffamine® ED-600 bzw. Jeffamine® XTJ-501 (jeweils Fa. Huntsman), aliphatische Polyetheramine, die aus einem mit Propylenoxid gepfropften Polyethylenglykol aufgebaut sind und eine mittlere Molmasse von 900 aufweisen, wie z. B. Jeffamine® ED-900 (Fa. Huntsman), aliphatische Polyetheramine, die aus einem mit Propylenoxid gepfropften Polyethylenglykol aufgebaut sind und eine mittlere Molmasse von 2000 aufweisen, wie z. B. Jeffamine® ED-2003 (Fa. Huntsman), difunktionelle, primäre Polyetheramin, hergestellt durch Aminierung eines mit Propylenoxid gepfropften Diethylenglykols mit einer mittleren Molmasse von 220 wie z. B. Jeffamine® HK-511 (Fa. Huntsman), aliphatische Polyetheramine auf der Basis eines Copolymers aus Poly(tetramethylenetherglykol) und Polypropylenglykol mit einer mittleren Molmasse von 1000 wie z. B. Jeffamine® XTJ-542 (Fa. Huntsman), aliphatische Polyetheramine auf der Basis eines Copolymers aus Poly(tetramethylenetherglykol) und Polypropylenglykol mit einer mittleren Molmasse von 1900 wie z. B. Jeffamine® XTJ-548 (Fa. Huntsman), aliphatische Polyetheramine auf der Basis eines Copolymers aus Poly(tetramethylenetherglykol) und Polypropylenglykol mit einer mittleren Molmasse von 1400 wie z. B. Jeffamine® XTJ-559 (Fa. Huntsman), Polyethertriamine auf der Basis eines mit Butylenoxid gepfropften mindestens dreiwertigen Alkohols mit einer mittleren Molmasse von 400 wie z. B. Jeffamine® XTJ-566 (Fa. Huntsman), aliphatische Polyetheramine, hergestellt durch Aminierung von mit Butylenoxid aufgepfropften Alkoholen mit einer mittleren Molmasse von 219 wie z. B. Jeffamine® XTJ-568 (Fa. Huntsman), Polyetheramine auf der Basis von Pentaerythrit und Propylenoxid mit einer mittleren Molmasse von 600 wie z. B. Jeffamine® XTJ-616 (Fa. Huntsman), Polyetheramine auf der Basis von Triethylenglykol mit einer mittleren Molmasse von 148, z. B. Jeffamine® EDR-148 (Fa. Huntsman), difunktionelle, primäre Polyetheramine, hergestellt durch Aminierung eines mit Propylenoxid gepfropften Ethylenglykols mit einer mittleren Molmasse von 176 wie z. B. Jeffamine® EDR-176 (Fa. Huntsman) sowie Polyetheramine, hergestellt durch Aminierung von Poly-Tetrahydrofuran (Poly-THF) mit einer mittleren Molmasse von 250, z. B. PolyTHF-Amin 350 (Fa. BASF SE) und Mischungen dieser Amine;
- Polyamidoamine (Amidopolyamine), die durch die Reaktion von dimeren Fettsäuren (z. B. dimere Linolsäure) mit niedermolekularen Polyaminen wie Diethylentriamin, 1-(3-Aminopropyl)-3-aminopropan oder Triethylentetramin oder anderen Diaminen wie den zuvor genannten aliphatischen oder cycloaliphatischen Diaminen erhältlich sind;
- Addukte, die durch Umsetzung von Aminen, insbesondere Diaminen, mit einem Unterschuss an Epoxidharz bzw. Reaktivverdünner erhältlich sind, wobei man vorzugsweise solche Addukte einsetzt, worin etwa 5 bis 20 % der Epoxidgruppen mit Aminen, insbesondere Diaminen, umgesetzt worden sind;
- Phenalkamine, wie aus der Epoxidchemie bekannt;
- Mannichbasen, welche z. B. durch Kondensation von Polyaminen, vorzugsweise Diethylentriamin, Triethylentetramin, Isophorondiamin, 2,2,4- bzw. 2,4,4-Trimethylhexamethylendiamin, 1,3- und 1,4-Bis(aminomethyl)cyclohexan mit Aldehyden, vorzugsweise Formaldehyd und ein- oder mehrwertigen Phenolen mit mindestens einer aldehydreaktiven Kernstelle, z. B. die verschiedenen Kresole und Xylenole, p-tert.-Butylphenol, Resorcin, 4,4'-Dihydroxydiphenylmethan, 4,4'-Dihydroxydiphenyl-2,2-propan, vorzugsweise aber Phenol, hergestellt werden;
sowie Mischungen der vorgenannten Aminhärter, insbesondere Mischungen von difunktionellen Aminen aus der Gruppe der aliphatischen, cycloaliphatischen und aromatischen Amine mit den vorgenannten Polyetheraminen.

Bevorzugte aminische Härter sind aliphatische Polyamine, insbesondere 2,2-Dimethylpropylendiamin, aromatische Diamine, insbesondere m-Xylylendiamin (MXDA) und cycloaliphatische Diamine, insbesondere Isophorondiamin, N-Cyclohexylpropylen-1,3-diamin und 4,4'-Diaminodicyclohexylmethan (Dicykan). Bevorzugt sind auch difunktionelle oder trifunktionelle primäre Polyetheramine auf der Basis von Polypropylenglykol, wie z. B. Jeffamine® D 230 oder Jeffamine® T 403. Besonders bevorzugt sind Polyamine, bei denen eine hohe Beweglichkeit und eine geringe sterische Hinderung rund um die Aminogruppe vorherrschen, z.B. 4,9-Dioxadodecan-1,12-diamin, 4,7,10-Trioxatridecan-1,13-diamin, PolyTHF Amin 350 (BASF SE).

Bevorzugt sind auch Mischungen der als bevorzugt genannten Amine, beispielsweise Mischungen, die 2,2-Dimethylpropylenamin und Isophoronamin enthalten.

Zu den alkoholischen Härtern zählen vor allem niedermolekulare und höhermolekulare aliphatische und cycloaliphatische Alkohole. Alkoholische Härter vernetzen zu Carbonatpolymeren durch Reaktion der primären oder sekundären Alkoholfunktionen mit den 1,3-Dioxolan-2-on-Gruppen unter Ausbildung von Diestern der Kohlensäure. Bevorzugte alkoholische Härter weisen im Mittel wenigstens zwei primäre oder sekundäre Hydroxygruppen pro Molekül, z. B. zwei, drei oder vier primäre oder sekundäre Hydroxygruppen pro Molekül, auf. Geeignete niedermolekulare alkoholische Härter sind z. B. 1,4-Butandiol, Ethylenglykol, Diethylenglykol, Triethylenglykol, Neopentylglykol, 1,3-Propandiol, 1,5-Pentandiol, 1,6-Hexandiol, Glycerin, Diglycerin, Pentaerythrit, Dipentaerythrit, Zuckeralkohole wie Sorbit und Mannit.

Geeignete alkoholische Härter sind auch höhermolekulare, polymere Polyole, beispielsweise Polyesterpolyole, Polycarbonatpolyole, Polyetherpolyole, Polyacrylatpolyole und Polyvinylalkohole. Geeignete polymere Polyolhärter weisen vorzugsweise eine mittlere OH-Funktionalität von wenigstens 1,5 mol und speziell wenigstens 1,8, z. B. im Bereich von 1,5 bis 10 und insbesondere im Bereich von 1,8 bis 4, auf. Unter der mittleren OH-Funktionalität versteht man die mittlere Anzahl OH-Gruppen je Polymerkette. Typische polymere Polyolkomponenten weisen vorzugsweise ein zahlenmittleres Molekulargewicht von etwa 250 bis 50000 g/mol, vorzugsweise von etwa 500 bis 10000 g/mol auf. Vorzugsweise sind wenigstens 50 mol-% der in der polymeren Polyolkomponente enthaltenen Hydroxylgruppen primäre Hydroxylgruppen.

Vorzugsweise handelt es sich bei Polyesterpolyolen um lineare oder verzweigte polymere Verbindungen mit Estergruppen im Polymerrückgrat, die an den Enden der Polymerkette freie Hydroxylgruppen aufweisen. Vorzugsweise handelt es sich hierbei um Polyester, die durch Polykondensation von zweiwertigen Alkoholen mit zweiwertigen Carbonsäuren, gegebenenfalls in Gegenwart höherwertiger Alkohole (z. B. 3, 4, 5 oder 6-wertiger Alkohole) und/oder höherwertiger Polycarbonsäuren, erhalten werden. Anstelle der freien Di- bzw. Polycarbonsäuren können auch die entsprechenden Di- bzw. Polycarbonsäureanhydride oder entsprechende Di- bzw. Polycarbonsäureester von niederen Alkoholen oder deren Gemische zur Herstellung der Polyesterpolyole verwendet werden. Die Di- bzw. Polycarbonsäuren können aliphatisch, cycloaliphatisch, araliphatisch, aromatisch oder heterocyclisch sein, weisen vorzugsweise 2 bis 50 und insbesondere 4 bis 20 C-Atome auf und können gegebenenfalls, z. B. durch Halogenatome, substituiert und/oder ungesättigt sein. Als Beispiele hierfür seien genannt: Korksäure, Azelainsäure, Phthalsäure, Isophthalsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Alkenylbernsteinsäure, Fumarsäure und dimere Fettsäuren. Für die Herstellung der Polyesterpolyole kommen als Diole insbesondere aliphatische und cycloaliphatische Diole mit vorzugsweise 2 bis 40 und insbesondere 2 bis 20 C-Atomen in Betracht, z. B. Ethylenglykol, Propan-1,2-diol, Propan-1,3-diol, Butan-1,3-diol, Butan-1,4-diol, Buten-1,4-diol, Butin-1,4-diol, Pentan-1,5-diol, Neopentylglykol, Bis-(hydroxymethyl)cyclohexane wie 1,4-Bis-(hydroxymethyl)cyclohexan, 2-Methylpropan-1,3-diol, Methylpentandiole, ferner Diethylenglykol, Triethylenglykol, Tetraethylenglykol, Polyethylenglykol, Dipropylenglykol, Polypropylenglykol, Dibutylenglykol und Polybutylenglykole. Bevorzugt sind Alkohole der allgemeinen Formel HO-(CH₂)ₓ-OH, wobei x eine Zahl von 2 bis 20, bevorzugt eine gerade Zahl von 2 bis 12 ist. Beispiele hierfür sind Ethylenglykol, Butan-1,4-diol, Hexan-1,6-diol, Octan-1,8-diol und Dodecan-1,12-diol. Weiterhin bevorzugt sind Neopentylglykol und Pentan-1,5-diol.

Geeignet als alkoholische Härter sind auch Polyesterpolyole auf Lacton-Basis, wobei es sich um Homo- oder Mischpolymerisate von Lactonen, bevorzugt um endständige Hydroxylgruppenaufweisende Anlagerungsprodukte von Lactonen an geeignete difunktionelle Startermoleküle handelt. Als Lactone kommen bevorzugt solche in Betracht, die sich von Verbindungen der allgemeinen Formel HO-(CH₂)_{z}-COOH ableiten, wobei z eine Zahl von 1 bis 20 ist und ein H-Atom einer Methyleneinheit auch durch einen C₁- bis C₄-Alkylrest substituiert sein kann. Beispiele sind ε-Caprolacton, β-Propiolacton, γ-Butyrolacton und/oder Methyl-ε-caprolacton sowie deren Gemische. Geeignete Startermoleküle sind z. B. die vorstehend als Aufbaukomponente für die Polyesterpolyole genannten niedermolekularen zweiwertigen Alkohole. Die entsprechenden Polymerisate des ε-Caprolactons sind besonders bevorzugt. Auch niedere Polyesterdiole oder Polyetherdiole können als Starter zur Herstellung der Lacton-Polymerisate eingesetzt sein. Anstelle der Polymerisate von Lactonen können auch die entsprechenden, chemisch äquivalenten Polykondensate der den Lactonen entsprechenden Hydroxycarbonsäuren eingesetzt werden. Beispiele für geeignete Polyesterpolyole sind z. B. die aus Ullmanns Enzyklopädie der Technischen Chemie, 4. Auflage, Band 19, Seiten 62 bis 65 bekannten Polyesterpolyole.

Ferner kommen auch Polycarbonatpolyole, wie sie z. B. durch Umsetzung von Phosgen mit einem Überschuss von den als Aufbaukomponenten für die Polyesterpolyole genannten niedermolekularen Alkohole erhalten werden können, in Betracht.

Bei den Polyetherpolyolen handelt es sich insbesondere um Polyetherpolyole, die durch Polymerisation von Ethylenoxid, Propylenoxid, Butylenoxid, Tetrahydrofuran, Styroloxid oder Epichlorhydrin mit sich selbst, z. B. in Gegenwart von BF₃ oder durch Anlagerung dieser Verbindungen gegebenenfalls im Gemisch oder nacheinander, an bi- oder polyfunktionelle Startkomponenten mit reaktionsfähigen Wasserstoffatomen, wie Polyole oder polyfunktionelle Amine, z. B. Wasser, Ethylenglykol, Propan-1,2-diol, Propan-1,3-diol, 1,1-Bis-(4-hydroxyphenyl)propan, Trimethylolpropan, Glycerin, Sorbit, Ethanolamin oder Ethylendiamin, hergestellt werden können. Auch Succrosepolyether (siehe DE 1176358 und DE 1064938) sowie auf Formit oder Formose gestartete Polyether (siehe DE 2639083 und DE 2737951) kommen in Frage.

Geeignet sind ebenfalls Polyhydroxyolefine, bevorzugt solche mit 2 endständigen Hydroxylgruppen, z. B. α-ω-Dihydroxypolybutadien.

Geeignet sind ebenfalls Polyhydroxypolyacrylate, wobei die Hydroxylgruppen seitenständig oder endständig angeordnet sein können. Beispiel hierfür sind α,ω-Dihydroxypoly(meth)acrylester, die durch Homo- oder Copolymerisation von Alkylestern der Acrylsäure und/oder der Methacrylsäure in Gegenwart von OH-Gruppen-enthaltenden Reglern wie Mercaptoethanol oder Mercaptopropanol und anschließende Umesterung mit einem niedermolekularen Polyol, beispielsweise einem Alkylenglykol wie Butandiol, erhältlich sind. Solche Polymere sind beispielsweise aus der EP-A 622 378 bekannt. Beispiel hierfür sind weiterhin Polymere, die durch Copolymerisation von Alkylestern der Acrylsäure und/oder der Methacrylsäure mit Hydroxyalkylestern ethylenisch ungesättigter Carbonsäure wie Hydroxyethylacrylat, Hydroxypropylacrylat, Hydroxybutylacrylat, Hydroxyethylmethacrylat, Hydroxypropylmethacrylat oder Hydroxybutylmethacrylat erhältlich sind.

Geeignet sind auch Polyvinylalkohole, die vorzugsweise durch vollständige oder teilweise Verseifung von Polyvinylestern, insbesondere Polyvinylacetat, erhalten werden können. Sofern die Polyvinylester, bevorzugt Polyvinylacetat, teilverseift vorliegen, liegen bevorzugt höchstens 50 bis 95 % der Estergruppen verseift als Hydroxylgruppen vor. Sofern die Polyvinylester, bevorzugt Polyvinylacetat, vollständig verseift vorliegen, liegen im Allgemeinen mehr als 95 % bis zu 100 % der Estergruppen verseift als Hydroxylgruppen vor.

Unter den höhermolekularen, polymeren Polyolen bevorzugte alkoholische Härter sind insbesondere Polyacrylatpolyole, diese sind beispielsweise unter dem Markennamen Joncryl® der Fa. BASF SE erhältlich, z. B. Joncryl® 945.

Geeignete Härter sind auch Aminosäuren, z. B. Lysin, Arginin, Glutamin und Asparagin und deren Stereoisomere und deren Mischungen.

Selbstredend können auch Mischungen unterschiedlicher Härter eingesetzt werden, z. B. Mischungen eines oder mehrerer aminischer Härter mit einem oder mehreren alkoholischen Härtern, Mischungen eines oder mehrerer aminischer Härter mit einer oder mehreren Aminosäuren oder Mischungen eines oder mehrerer alkoholischer Härter mit einer oder mehreren Aminosäuren.

In den erfindungsgemäßen Bindemittelzusammensetzungen beträgt die Gesamtmenge an Härtern vorzugsweise 0,1 Gew.-% bis 50 Gew.-%, häufig 0,5 bis 40 Gew.-% und insbesondere 1 bis 30 Gew.-%, bezogen auf die Gesamtmenge an Exovinylen-cyclocarbonatverbindungen plus eingesetzten Härtern.

Die Härtung der Bindemittelzusammensetzung kann thermisch durch Erwärmen des Gemischs aus erfindungsgemäßem Polymer und Härter auf eine Temperatur oberhalb der Mischtemperatur erfolgen. Die Härtung kann auch bei niedrigeren Temperaturen erfolgen. Typischerweise erfolgt die Härtung der erfindungsgemäßen Bindemittelzusammensetzungen bei Temperaturen im Bereich von -10°C bis 150 °C, vorzugsweise im Bereich von 0 bis 100 °C und insbesondere im Bereich von 10 bis 70 °C. Besonders vorteilhaft ist die Härtung bei Temperaturen von 20-30°C. Welche Temperatur geeignet ist, hängt von den jeweiligen Härtern und der gewünschten Härtungsgeschwindigkeit ab und kann im Einzelfall vom Fachmann beispielsweise anhand einfacher Vorversuche ermittelt werden. Im unteren Temperaturbereich (5 bis ca. 35 C), der ja der meist vorherrschenden Umgebungstemperatur entspricht, reicht es selbstverständlich aus, erfindungsgemäßes Polymer und Härter zu mischen. Alternativ erfolgt die Härtung vorzugsweise mikrowelleninduziert.

Die 2K-Bindemittelzusammensetzungen können auch einen oder mehrere geeignete Katalysatoren für die Aushärtung enthalten, die sich in bekannter Weise nach der Art der reaktiven funktionellen Gruppen F richten. Die Katalysatoren werden, sofern erwünscht, in Anteilen von 0,01 Gew.-% bis etwa 10 Gew.-%, bezogen auf das Gesamtgewicht der erfindungsgemäßen Polymere mit funktionellen Alkyliden-1,3-dioxolan-2-on-Gruppen der Formel I und des Härters, eingesetzt. In einer Ausgestaltung werden keine Katalysatoren benötigt, insbesondere bei Härtern, die als funktionelle Gruppen Aminogruppen aufweisen, d.h., der Gehalt an Katalysatoren in der Zusammensetzung beträgt dann weniger als 0,01 Gew.-%. Katalysatoren werden bevorzugt dann eingesetzt, wenn der Härter reaktive Gruppen F aufweist, die von Aminogruppen verschieden sind, insbesondere wenn der Härter Hydroxylgruppen aufweist.

Bevorzugt eingesetzte Katalysatoren sind basische Katalysatoren, besonders bevorzugt organische Amine und organische Phosphine. Unter den organischen Aminen sind Amidinbasen, wie beispielsweise 1,8-Diazabicyclo[5.4.0]undec-7-en (DBU) und 1,5-Diazabicyclo[4.3.0]non-5-en (DBN), Mono-C₁-C₆-alkyl-, Di-C₁-C₆-alkyl- und Tri-C₁-C₆-alkylamine, insbesondere Triethylamin und tert.-Butylamin, bevorzugt. Unter den organischen Phosphinen sind Trialkylphosphine und Triarylphosphine bevorzugt, beispielsweise Tri-n-butylphosphin und Triphenylphosphin. Die Katalysatoren können selbstverständlich auch als Mischungen eingesetzt werden, gegebenenfalls in Kombination mit Tri-C₁-C₆-alkylammoniumhalogeniden und Kupfersalzen, zum Beispiel Triphenylphosphin in Kombination mit einem Tri-C₁-C₆-alkylammoniumhalogenid und einem Kupfersalz, z. B. Kupfer(I)-chlorid, Kupfer(I)-bromid, Kupfer(II)-chlorid oder Kupfer(II)-sulfat.

Neben den vorgenannten Bestandteilen kann die 2K-Bindemittelzusammensetzung die hierfür üblichen Additive enthalten. Die Wahl geeigneter herkömmlicher Additive für die erfindungsgemäße Zusammensetzung hängt vom jeweiligen Verwendungszweck der 2K-Bindemittelzusammensetzung ab und kann im Einzelfall vom Fachmann bestimmt werden.

Geeignete Additive umfassen beispielsweise Antioxidantien, UV-Absorber/Lichtstabilisatoren, Metalldeaktivatoren, Antistatika, Verstärkungsstoffe, Füllstoffe, Antifoggingmittel, Treibmittel, Biozide, Weichmacher, Gleitmittel, Emulgatoren, Farbmittel, Pigmente, Rheologiemittel, Schlagzähigkeitsverbesserer, Adhäsionsregulatoren, optische Aufheller, Flammschutzmittel, Antitropfmittel, Nukleierungsmittel, Netzmittel, Verdicker, Schutzkolloide, Entschäumer, Tackifier, Lösungsmittel und Reaktivverdünner sowie Gemische davon.

Füllstoffe können organischer und anorganischer Natur sein; bevorzugte anorganische Füllstoffe liegen in Form von Plättchen vor, die sich zu Schichten mit verstärkter Barrierewirkung gegenüber Flüssigkeiten und Gasen ausrichten lassen. Beispiel sind Schichtsilikate wie Montmorillonit und Hectorit, wie sie z.B. in der WO 2011/089089, WO 2012/175427 oder in der WO 2012/175431 beschrieben sind. Bevorzugt sind Schichtsilikate, die ein Aspektverhältnis von mindestens 50, mindestens 400, oder mindestens 1000 und insbesondere größer oder gleich 10000 aufweisen. Die Schichtdicke ist z.B. ungefähr 1 nm. Die Schichtsilikate können natürlichen oder synthetischen Ursprungs sein. Geeignete Schichtsilikate sind z.B. Montmorillonit, Bentonit, Kaolinit, Mica, Hectorit, Fluorohectorit, Saponit, Beidellit, Nontronit, Stevensite, Vermiculit, Fluorovermiculit, Halloysit, Volkonskoit, Suconit, Magadit, Sauconit, Stibensit, Stipulgit, Attapulgit, Illit, Kenyait, Smectit, Allevardit, Muscovit, Palygorskit, Sepiolit, Silinait, Grumantit, Revdit, Zeolit, Fuller's earth, natürlicher oder synthetischer Talk oder Mica, oder Permutit. Besonders bevorzugt sind Montmorillonit (Aluminiummagnesiumsilikat), Hectorit (Magnesiumlithiumsilikat), synthetisches Fluorohectorit und exfolierte, organisch modifizierte Smectite. Die Schichtsilikate können modifiziert oder unmodifiziert sein. Bevorzugt sind kationisch modifizierte Schichtsilikate. Kationisch modifiziert bedeutet, dass anorganische Kationen des Schichtsilikats zumindest zum Teil gegen organische Kationen ausgetauscht sind, z.B. durch ein Ionenaustauschverfahren. Organische Kationen sind organische Verbindungen, welche mindestens eine kationische Gruppe aufweisen, z.B. quaternäre Ammoniumgruppe, Phosphoniumgruppe, Pyridiniumgruppe oder ähnliche oder ein kationisches Aminsalz.

Die gegebenenfalls verwendeten Lichtstabilisatoren/UV-Absorber, Antioxidantien und Metalldeaktivatoren weisen vorzugsweise eine hohe Migrationsstabilität und Temperaturbeständigkeit auf. Sie sind beispielsweise aus den Gruppen a) bis t) ausgewählt. Die Verbindungen der Gruppen a) bis g) und i) stellen Lichtstabilisatoren/UV-Absorber dar, während Verbindungen j) bis t) als Stabilisatoren wirken.
a) 4,4-Diarylbutadiene,
b) Zimtsäureester,
c) Benzotriazole,
d) Hydroxybenzophenone,
e) Diphenylcyanacrylate,
f) Oxamide,
g) 2-Phenyl-1,3,5-triazine,
h) Antioxidantien,
i) Nickelverbindungen,
j) sterisch gehinderte Amine,
k) Metalldesaktivatoren,
l) Phosphite und Phosphonite,
m) Hydroxylamine,
n) Nitrone,
o) Aminoxide,
p) Benzofuranone und Indolinone,
q) Thiosynergisten,
r) Peroxid-zerstörende Verbindungen,
s) Polyamidstabilisatoren und
t) basische Costabilisatoren.

Das Zweikomponenten-Bindemittel ist vorzugsweise frei von Isocyanaten, d.h. er enthält vorzugsweise keine Isocyanatverbindungen als Härter. Das Zweikomponenten-Bindemittel liegt vorzugsweise entweder in Form einer Lösung in einem organischen Lösungsmittel vor oder es ist lösemittelfrei. Lösemittelfrei bedeutet, dass weniger als 5 Gew.%, besonders bevorzugt weniger als 2 Gew.% oder kein organisches Lösungsmittel oder Wasser enthalten ist.

Das erfindungsgemäße Zweikomponenten-Bindemittel ist in der Lage, in kurzer Zeit und insbesondere mit Aminhärtern bereits bei Raumtemperatur hohe Bindekräfte aufzubauen.

### Beispiele

Beispiel 1: Synthese eines Exovinylen-cyclocarbonat-dimethylacetals Die Herstellung erfolgt in zwei Stufen. 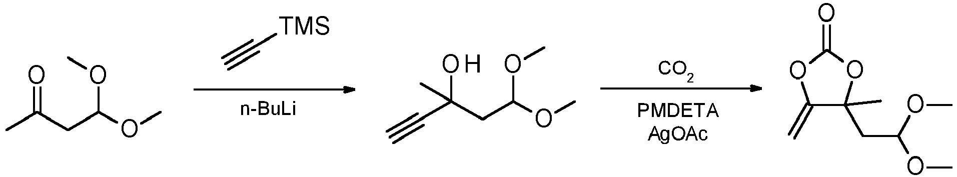
1.) Ethinylierung von 4,4-Dimethoxybutan-2-on:
   TMS-Acetylen (982 g, 10 mol) wird in THF (17 L, getrocknet über Molsieb) unter Argon vorgelegt und auf-68 °C abgekühlt. Unter Rühren wird innerhalb von 1 h *n*-Butyllithium (2,5 M in Hexan, 4 L) bei -68 °C zugetropft und 1 h nachgerührt. Innerhalb von 30 min wird dann das Keton (1,319 kg, 10 mol) bei -68°C bis -54°C zugetropft und in Anschluss 15 min nachgerührt. Danach wird auf 9°C erwärmt und Wasser (2,9 L) wird in einer Portion zugegeben. Dabei steigt die Temperatur auf ca. 17°C an. Das Reaktionsgemisch wird bei 45°C/8 Torr gründlich eingeengt. Durch GC-Analytik wird sichergestellt, dass kein TMS-geschütztes Produkt mehr enthalten ist. Der Rückstand wird in Diethylether (750 ml) aufgeschlämmt, filtriert und der Filtrationsrückstand nochmals mit Diethylether gewaschen. Das Filtrat wird im Vakuum eingeengt. Es bleiben ca. 1,2 kg Rohmaterial als braune Flüssigkeit. Durch Vakuumdestillation (5 mbar) werden daraus bei 64-68 °C ca. 1,1 kg (7 mol, 70%) ethinyliertes Produkt als farbloses Öl erhalten. Reinheit: >96% (GC-Flächen%)
2.) Ringschluss mit CO₂:
   Der in Stufe 1 erhaltene Acetylenalkohol (1233 g; 7,79 mol) wird in Acetonitril (1,2 L) vorgelegt und in einem Rührautoklaven mit PMDETA (Pentamethyldiethylentriamin; 138,9 g; 0,8 mol) und AgOAc (12,9 g; 0,078 mol) versetzt. Es werden 50 bar CO₂ aufgepresst und 2,5 h gerührt. Die Temperatur steigt bis auf 75°C. Das Reaktionsgemisch wird nach Abkühlen auf Raumtemperatur auf Normaldruck entspannt, filtriert und bei 100°C/5 mbar eingeengt. Es bleiben ca. 1,5 kg Rohmaterial als braune Flüssigkeit. Durch Vakuumdestillation bei 5 mbar werden daraus bei 114-115°C ca. 1,39 kg des Carbonats als orangefarbenes Öl erhalten, welches (ggfs. nach Zusatz einiger Impfkristalle) über Nacht durchkristallisiert. Die Kristallmasse wird mit Cyclohexan (1,34 L) verrührt, abgesaugt und der Rückstand nochmals mit Cyclohexan (0,45 L) nachgewaschen. Nach Trocknen im Vakuum werden 1,29 kg (6,38 mol, 64%) fast farbloser Feststoff erhalten.
   Reinheit: >99% (GC-Flächen%)

### Beispiel 2: Umacetalisierung mit 1,6-Hexandiol

In einem 3- Halskolben mit Rührer Thermometer und Vakuumanschluss wurden 30 g der Verbindung aus Beispiel 1, 22,8 g 1,6 Hexandiol, 0,5 g p-Toluolsulfonsäure in 30 g Acetonitril suspendiert und auf 45°C erwärmt. Es wurde Vakuum gezogen und das Lösungsmittel abdestilliert, insgesamt 8 h bei 45°C und 40 h bei Raumtemperatur destilliert. Dann wurde 3 mal mit 150 ml VE-Wasser gewaschen und abdekantiert, und im Vakuum getrocknet.
Ausbeute : 40 g gelbe, viskose Flüssigkeit
Nullviskosität bei 23°C: 2 Pa s

### Beispiel 3: Umacetalisierung mit 1,4-Butandiol

In einem 3- Halskolben mit Rührer Thermometer und Vakuumanschluss wurden 100 g der Verbindung aus Beispiel 1, 57,9 g 1,4-Butandiol in 50 g Acetonitril gelöst. 1,5 g p-Toluolsulfonsäure wurden in 10 g Acetonitril gelöst und dem Ansatz zugesetzt. Es wurde das Lösungsmittel abdestilliert und 40 h bei Raumtemperatur Vakuum gezogen und weiterhin 8 h bei 45° C destilliert. Dann wurde 3 mal mit 50 ml VE-Wasser gewaschen und abdekantiert, und im Vakuum getrocknet.
Ausbeute : 100 g braune , viskose Flüssigkeit

### Beispiel 4: Umacetalisierung mit Triethylenglykol

In einem 3- Halskolben mit Rührer Thermometer und Vakuumanschluss wurden 100 g der Verbindung aus Beispiel 1, 96,5 g Triethylenglykol (über Molsieb getrocknet), 1,36 g p-Toluolsulfonsäure in 50 g Acetonitril suspendiert und auf 45°C erwärmt. Es wurde Vakuum gezogen und das Lösungsmittel abdestilliert, weiterhin 8 h bei 45°C und 40 h bei Raumtemperatur destilliert. Dann wurde 3 mal mit 150 ml VE-Wasser gewaschen und abdekantiert, und im Vakuum getrocknet.
Ausbeute : 153 g braune , viskose Flüssigkeit

### Beispiel 5: Umacetalisierung mit 1,6 Hexandiol (gepuffert, getrocknet aufgearbeitet)

In einem 3-Halskolben mit Rührer Thermometer und Vakuumanschluss wurden 100 g der Verbindung aus Beispiel 1, 75,98 g 1,6 Hexandiol, 1,7 g p-Toluolsulfonsäure in 100 g Acetonitril suspendiert und auf 45°C erwärmt. Es wurde Vakuum gezogen und das Lösungsmittel abdestilliert , insgesamt 8 h bei 45°C und 12 h bei Raumtemperatur destilliert. Dann wurde in 200 g Methyl-tert.-butylether aufgenommen, 3 mal mit 50 ml Pufferlösung pH7 gewaschen, mit MgSO4 getrocknet, filtriert und das Lösungsmittel abrotiert.
Ausbeute : 131 g gelbe, viskose Flüssigkeit

### Beispiel 6: Umacetalisierung mit 1,6 Hexandiol (mit heterogenem Katalysator)

In einem 3- Halskolben mit Rührer Thermometer und Vakuumanschluss wurden 20 g der Verbindung aus Beispiel 1, 15,2 g 1,6-Hexandiol , 2,5g Amberlyst 15 trocken, sauer (Sigma-Aldrich) in 20 ml Acetonitril suspendiert und auf 45°C erwärmt. Es wurde Vakuum gezogen und das Lösungsmittel abdestilliert , insgesamt 8 h bei 45° C und 40 h bei Raumtemperatur destilliert. Dann wurde in 100 g Methyl-tert.-butylether (MTBE) aufgenommen, der heterogene Katalysator abfiltriert und das Lösungsmittel abrotiert.
Ausbeute : 19 g braune, viskose Flüssigkeit

### Beispiel 7: Umacetalisierung mit Poly-THF

In einem 3-Halskolben mit Rührer Thermometer und Vakuumanschluss wurden 20 g der Verbindung aus Beispiel 1, 32 g poly-THF 250, 0,34 g p-Toluolsulfonsäure in 20 g Acetonitril gelöst und auf 45°C erwärmt. Es wurde Vakuum gezogen und das Lösungsmittel abdestilliert, danach insgesamt 8 h bei 45°C und 40 h bei Raumtemperatur destilliert. Dann wurde in 100g Methyl-tert.-butylether aufgenommen, 3 mal mit 50 ml Pufferlösung pH7 gewaschen, mit MgSO4 getrocknet filtriert und das Lösungsmittel abrotiert.
Ausbeute : 37 g gelbe, viskose Flüssigkeit

### Beispiel 8: Umacetalisierung mit 3-Methylpentandiol

In einem 3-Halskolben mit Rührer Thermometer und Vakuumanschluss wurden 50 g der Verbindung aus Beispiel 1, 38 g 3-Methylpentandiol-1,5, 0,85 g p-Toluolsulfonsäure in 50 g Acetonitril suspendiert und auf 45°C erwärmt. Es wurde Vakuum gezogen und das Lösungsmittel abdestilliert, insgesamt 8 h bei 45° und 12 h bei Raumtemperatur destilliert. Dann wurde in 100 g Methyl-tert.-butylether aufgenommen, 3 mal mit 50 ml Pufferlösung pH7 gewaschen, mit MgSO4 getrocknet, filtriert und das Lösungsmittel abrotiert.
Ausbeute: 50,5 g gelbe, viskose Flüssigkeit
Nullviskosität bei 23°C: 40 Pa s

### Beispiel 9: Umacetalisierung mit 1,6-Cyclohexandimethanol

In einem 3-Halskolben mit Rührer Thermometer und Vakuumanschluss wurden 20 g der Verbindung aus Beispiel 1, 18,5 g 1,6-Cyclohexandimethanol, 0,34 g p-Toluolsulfonsäure in 20 g Acetonitril suspendiert und auf 45°C erwärmt. Es wurde Vakuum gezogen und das Lösungsmittel abdestilliert, insgesamt 8 h bei 45° und 12 h bei Raumtemperatur destilliert. Dann wurde in 100 g Methyl-tert.-butylether aufgenommen, 3 mal mit 50 ml Pufferlösung pH7 gewaschen, mit MgSO4 getrocknet, filtriert und das Lösungsmittel abrotiert.
Ausbeute: 27 g gelbe, hochviskose Flüssigkeit
Nullviskosität bei 23°C: 425 Pa s

### Beispiel 10: Umacetalisierung mit Butandiol in Methanol

In einem 3-Halskolben mit Rührer, Thermometer und Vakuumanschluss wurden 30 g der Verbindung aus Beispiel 1, 17,4 g 1,4-Butandiol in 30 g Methanol gelöst. 0,51 g Methansulfonsäure wurden in 10 g Methanol gelöst und dem Ansatz zugesetzt. Es wurde das Lösungsmittel abdestilliert und 40 h bei Raumtemperatur Vakuum gezogen und weiterhin 8 h bei 45° C destilliert. Dann wurde 3 mal mit 150 ml kaltem VE-Wasser und 3 mal mit 150 ml warmen VE-Wasser gewaschen, abdekantiert und im Vakuum getrocknet.
Ausbeute 14 g braune , viskose Flüssigkeit

### Beispiel 11: Umacetalisierung mit 1,8-Oktandiol

In einem 3-Halskolben mit Rührer, Thermometer und Vakuumanschluss wurden 300 g der Verbindung aus Beispiel 1, 282 g 1,8-Oktandiol, 2,5 g p-Toluolsulfonsäure in 410 g Acetonitril suspendiert und auf 55°C Außentemperatur erwärmt. Es wurde Vakuum gezogen und das Lösungsmittel abdestilliert, dabei sollte die Innentemperatur nicht unter 50°C fallen (insgesamt 8 h). Dann 15 h bei Raumtemperatur Vakuum gezogen und nochmals 8 h bei 50°C Außentemperatur entgast. Dann wurde in 400 g Methyl-tert.-butylether aufgenommen, 3 mal mit 150 ml Pufferlösung pH7 gewaschen, mit MgSO4 getrocknet, filtriert und das Lösungsmittel abrotiert.
Ausbeute : 457 g rot-braune viskose Flüssigkeit
Nullviskosität bei 23°C: 14 Pa s

### Beispiel 12: Umacetalisierung mit Diglycerin

In einem 3-Halskolben mit Rührer, Thermometer und Vakuumanschluss wurden 300 g der Verbindung aus Beispiel 1, 123,3 g Diglycerin (CAS.No 59113-36-9 Inovyn) in 100 g Methanol gelöst. 2,5 g p-Toluolsulfonsäure wurden in 10 g Methanol gelöst und dem Ansatz zugesetzt. Es wurde das Lösungsmittel abdestilliert und 8h bei Raumtemperatur destilliert. Dann wurde mit 150 ml Pufferlösung pH 8 ausgerührt, und in 300 ml Essigsäureethylester aufgenommen, 2 mal mit 150 ml Pufferlösung pH 7 ausgeschüttelt, mit MgSO4 getrocknet und das Lösungsmittel abdestilliert.
Ausbeute: 298 g sehr dunkle, sehr viskose Flüssigkeit
Nullviskosität bei 23°C: 4298 Pa s

### Beispiel 13 (Vergleich): Umacetalisierung mit einem Hydroxyalkylacrylat Copolymer (Acrylatpolymer als verbindende Gruppe)

In einem 3-Halskolben mit Rührer Thermometer und Vakuumanschluss wurden 7 g der Verbindung aus Beispiel 1 mit 22,8 g eines Hydroxyalkylacrylat Copolymers mit einer OH-Zahl von 85 mg KOH/g (Joncryl® 960, BASF) in 10 g Acetonitril gelöst. 0,07 g Methansulfonsäure wurden in 5 g Acetonitril gelöst und dem Ansatz zugesetzt. Es wurde das Lösungsmittel abdestilliert und 48 h bei Raumtemperatur Vakuum gezogen bis im IR-Spektrum keine OH-Bande mehr zu sehen war. Das Produkt ist sehr hochviskos und kaum mehr rührbar. Dann wurde 3 mal mit 50 ml VE-Wasser gewaschen und abdekantiert und im Vakuum getrocknet.
Ausbeute: 20 g gelbes, sehr viskoses Gel, nicht mehr rührbar

### Zweikomponenten-Beschichtungsstoffe

10 g des im Beispiel 3 beschriebenen Produktes wurden mit 2,0 g des Diamins 4,9-Dioxadodecan-1,12-diamin (DODA) als Härter abgemischt und das resultierende reaktive Zweikomponenten-Beschichtungsmittel wurde unmittelbar nach dem Mischen in einer Schichtdicke von 3 µm auf eine bedruckte 36 µm starke Polyesterfolie aufgetragen. Dann wurde in einem Kalander eine zweite 36 µm starke Polyesterfolie, unter einem Druck von 6,5 bar mit einer Kalandergeschwindigkeit von 5 m/min auf die Beschichtungsschicht kaschiert. Das resultierende Laminat wurde in 15 mm breite Streifen geschnitten und von diesen Streifen wurde nach 4 h die Schälfestigkeit bei Raumtemperatur (23 °C) ermittelt [N/15 mm]. Dazu bediente man sich einer Reißmaschine und führte die Schälfestigkeitsprüfung unter einem Reißwinkel von 90° durch (T-Test). Die Ergebnisse sind in Tabelle 1 wiedergegeben.

Analog wurden 10 g des in Beispiel 5 beschriebenen Produktes mit 3,15 g DODA als Härter abgemischt, verarbeitet und untersucht.

**Tabelle 1: Ergebnisse der Schälfestigkeitsmessungen**

| | Schälfestigkeit nach 4 h [N/15 mm] |
|---|---|
| 10 g Beispiel 3 abgemischt mit 2 g DODA | 3,2 |
| 10 g Beispiel 5 abgemischt mit 3,15 g DODA | 1,6 |

Eine Schälfestigkeit von größer 1 N nach 4 h ist für Anwendungen des Beschichtungsmittels, z.B. zur Herstellung von flexiblen Verpackungen ausreichend und baut sich in einer hinreichend kurzen Zeit auf, um industriell verwertbar zu sein.

## Patentansprüche

1. Verbindung mit zwei oder mehr Exovinylen-cyclocarbonateinheiten, wobei die Exovinylen-cyclocarbonateinheiten über mindestens eine organische, siloxanfreie Verbindungsgruppe miteinander verbunden sind, wobei die Verbindungsgruppe nicht direkt mit den Exovinylen-Doppelbindungen verbunden ist und wobei durch Polymerisation von (Meth)acrylmonomeren gebildete Verbindungsgruppen ausgenommen sind,
**dadurch gekennzeichnet, dass** die Verbindungsgruppe mindestens eine Acetalgruppe aufweist und es sich um Verbindungen der Formel (Vb) handelt 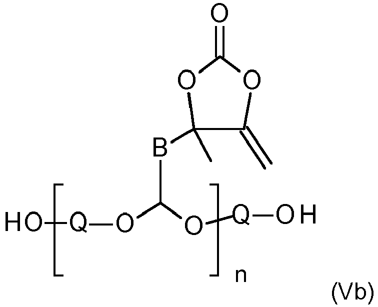 oder dass es sich um Verbindungen der Formel (Vc) handelt 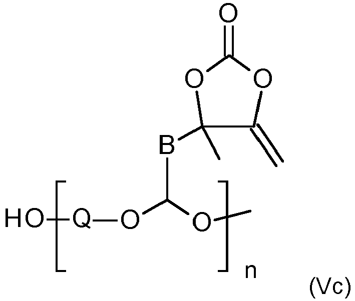 oder dass es sich um Verbindungen der Formel (IVb) handelt 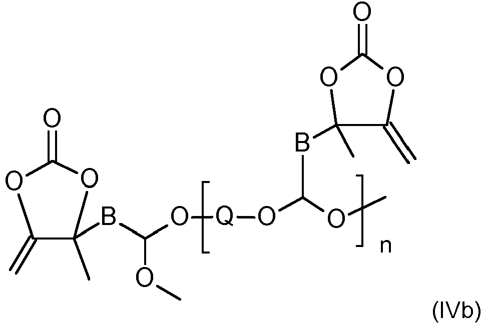 oder dass es sich um Verbindungen der Formel (VI) handelt 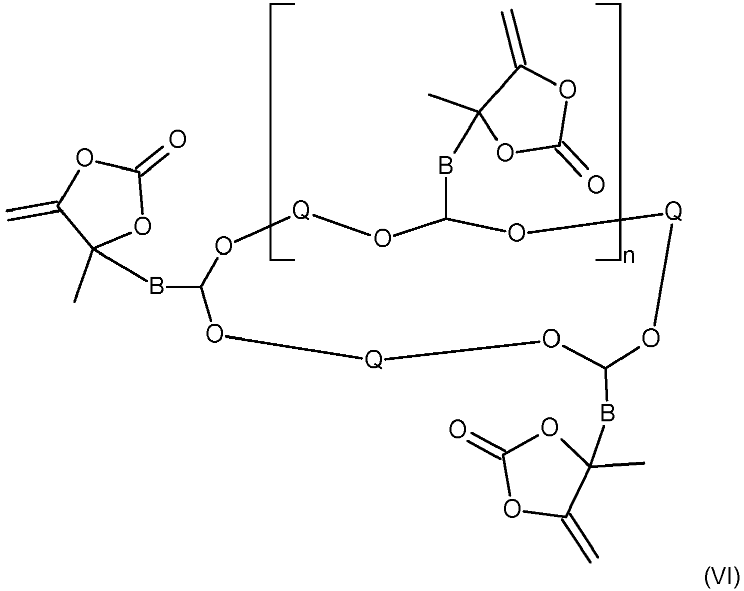
wobei B eine Alkylengruppe mit vorzugsweise 1 bis 6 C-Atomen bedeutet;
Q eine von einem Poylol abgeleitete Gruppe bedeutet, wobei ein Polyol ein Alkohol mit mindestens zwei Hydroxygruppen ist;
n in den Formeln (Vb) und (Vc) eine Zahl größer oder gleich 2, vorzugsweise 2 bis 10 ist,
n in Formel (IVb) eine Zahl größer oder gleich 1, vorzugsweise 1 bis 10, ist, und
n in Formel (VI) eine Zahl größer oder gleich 0, vorzugsweise 0 bis 10, ist.

2. Verbindung gemäß Anspruch 1, **dadurch gekennzeichnet, dass** es sich um Verbindungen der Formel (VIa) handelt 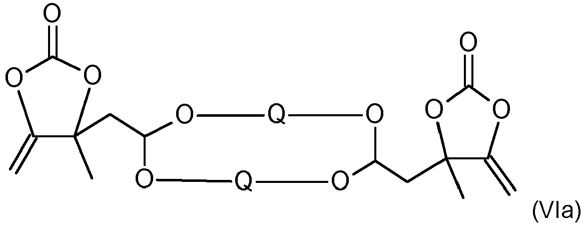 oder dass es sich um Verbindungen der Formel (IVc) handelt 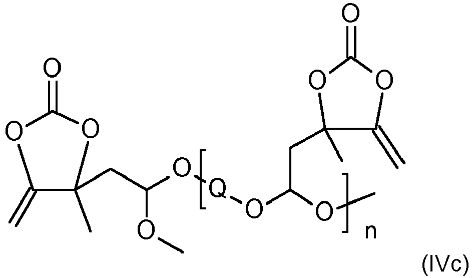 oder dass es sich um Verbindungen der Formel (Vd) handelt 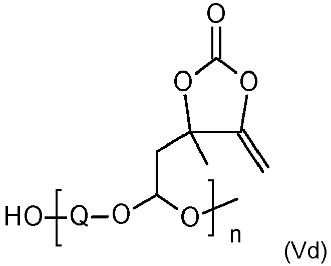 oder dass es sich um Verbindungen der Formel (Ve) handelt 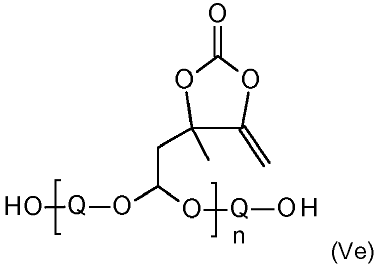
wobei -O-Q-O- in den Formeln VIa, IVc, Vd und Ve jeweils für den Rest eines zweiwertigen Alkohols steht, vorzugsweise ausgewählt aus Ethylenglykol, 1,3-Propandiol, 1,4-Butandiol, Diethylenglykol, Triethylenglykol, Neopentylglykol, 1,5-Pentandiol, 1,6-Hexandiol, 1,8-Octandiol, 3-Methyl-1,5-pentandiol, 1,4-bis-Hydroxymethyl-cyclohexan, 1,6-bis-Hydroxymethyl-cyclohexan, Glycerin, Diglycerin, Polyethylenglykol, Polypropylenglykol, Poly-THF, Pentaerythrit, Dipentaerythrit, Zuckeralkohole wie Sorbit und Mannit,
n in Formel (IVc) 1, 2 oder 3 ist, und
n in den Formeln (Vd) und (Ve) eine Zahl größer oder gleich 2, vorzugsweise 2 oder 3 ist.

3. Verfahren zur Herstellung einer Verbindung gemäß einem der Ansprüche 1 bis 2, wobei eine Verbindung, welche eine Exovinylen-cyclocarbonateinheit und mindestens eine Acetalgruppe aufweist, umgesetzt wird mit mindestens einer Verbindung ausgewählt aus der Gruppe bestehend aus Diolen und Polyolen.

4. Verfahren gemäß dem vorhergehenden Anspruch, **dadurch gekennzeichnet, dass** die Umsetzung unter Katalyse mit Lewissäuren oder mit Protonensäuren durchgeführt wird.

5. Verwendung einer Zwei-Komponentenzusammensetzung als Bindemittel für Lacke, Farben, Tinten, Baustoffe, Elastomere, Schäume, Fasern oder Partikel, wobei eine erste Komponente mindestens eine Verbindung gemäß einem der Ansprüche 1 bis 2 enthält und eine zweite Komponente mindestens einen mehrfunktionellen Härter enthält, welcher mindestens zwei funktionelle Gruppen aufweist ausgewählt aus der Gruppe bestehend aus primären Aminogruppen, sekundären Aminogruppen, Hydroxygruppen, Phosphingruppen, Phosphonatgruppen und Mercaptangruppen.

6. Verwendung gemäß dem vorhergehenden Anspruch, **dadurch gekennzeichnet, dass** die Zwei-Komponentenzusammensetzung mindestens einen Katalysator enthält für die Katalysierung der Reaktion der Exovinylen-cyclocarbonatgruppen mit den funktionellen Gruppen des Härters.

7. Verwendung einer Verbindung gemäß einem der Ansprüche 1 bis 2 als Bestandteil von Beschichtungsstoffen, Lacken, Farben, Tinten, Baustoffen, Elastomeren, Schäumen oder zur Bindung von Fasern und/oder Partikeln.

## Claims

1. A compound having two or more exovinylene cyclocarbonate units, where the exovinylene cyclocarbonate units are joined to one another via at least one organic, siloxane-free connecting group, where the connecting group is not bonded directly to the exovinylene double bonds and where connecting groups formed by polymerization of (meth)acrylic monomers are excluded,
wherein the connecting group has at least one acetal group and the compounds are of the formula (Vb) 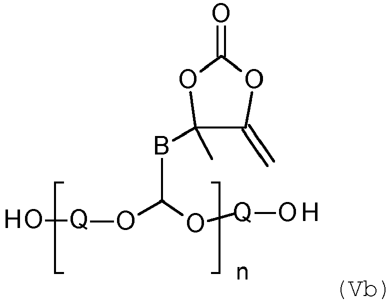 or wherein the compounds are of the formula (Vc) 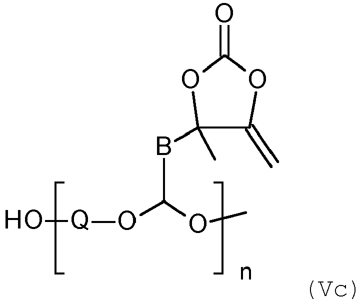 or wherein the compounds are of the formula (IVb) 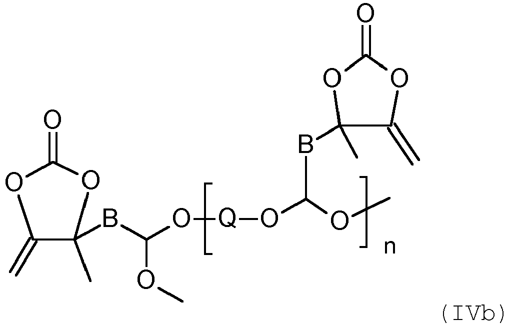 or wherein the compounds are of the formula (VI) 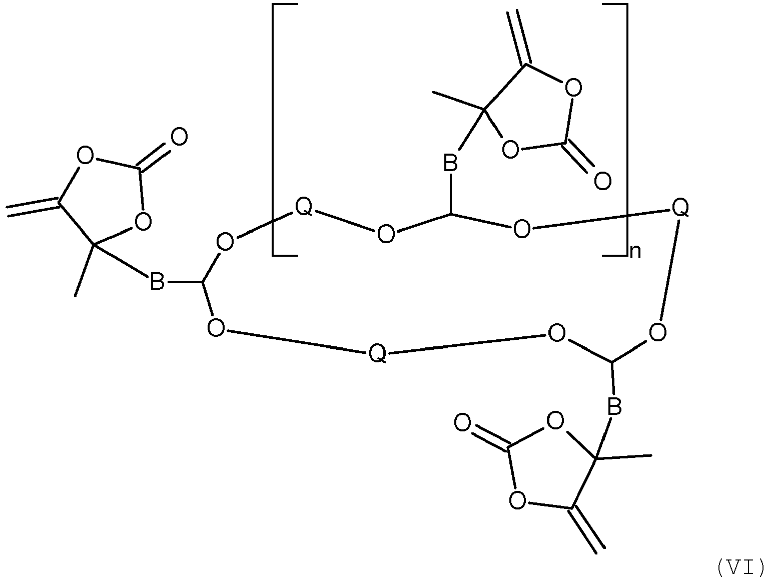
where B is an alkylene group having preferably 1 to 6 carbon atoms;
Q is a group derived from a polyol, a polyol being an alcohol having at least two hydroxyl groups;
n in the formulae (Vb) and (Vc) is a number not less than 2, preferably 2 to 10,
n in formula (IVb) is a number not less than 1, preferably 1 to 10, and
n in formula (VI) is a number not less than 0, preferably 0 to 10.

2. A compound according to claim 1, wherein the compounds are of the formula (VIa) 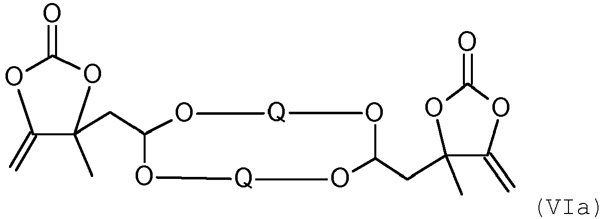 or wherein the compounds are of the formula (IVc) 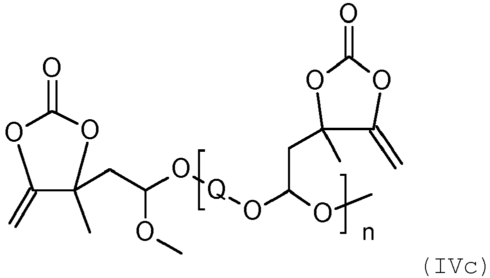 or wherein the compounds are of the formula (Vd) 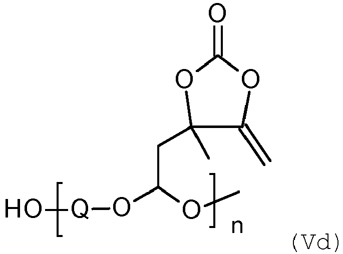 or wherein the compounds are of the formula (Ve) 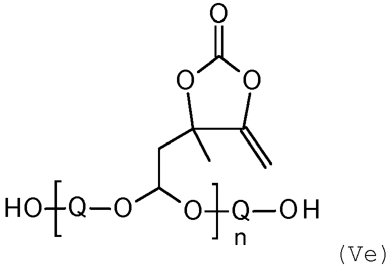
where -O-Q-O- in the formulae VIa, IVc, Vd and Ve in each case is the radical of a divalent alcohol, preferably selected from ethylene glycol, propane-1,3-diol, butane-1,4-diol, diethylene glycol, triethylene glycol, neopentyl glycol, pentane-1,5-diol, hexane-1,6-diol, octane-1,8-diol, 3-methylpentane-1,5-diol, 1,4-bis(hydroxymethyl)cyclohexane, 1,6-bis(hydroxymethyl)cyclohexane, glycerol, diglycerol, polyethylene glycol, polypropylene glycol, poly-THF, pentaerythritol, dipentaerythritol, sugar alcohols such as sorbitol and mannitol,
n in formula (IVc) is 1, 2 or 3, and
n in the formulae (Vd) and (Ve) is a number not less than 2, preferably 2 or 3.

3. A process for preparing a compound according to either of claims 1 and 2, wherein a compound having an exovinylene cyclocarbonate unit and at least one acetal group is reacted with at least one compound selected from the group consisting of diols and polyols.

4. The process according to the preceding claim, wherein the reaction is conducted under catalysis by Lewis acids or by protic acids.

5. The use of a two-component composition as binder for varnishes, paints, inks, building materials, elastomers, foams, fibers or particles, wherein a first component comprises at least one compound according to either of claims 1 and 2 and a second component comprises at least one polyfunctional hardener having at least two functional groups selected from the group consisting of primary amino groups, secondary amino groups, hydroxyl groups, phosphine groups, phosphonate groups and mercaptan groups.

6. The use according to the preceding claim, wherein the two-component composition comprises at least one catalyst for the catalysis of the reaction of the exovinylene cyclocarbonate groups with the functional groups of the hardener.

7. The use of a compound according to either of claims 1 and 2 as a constituent of coating compositions, varnishes, paints, inks, building materials, elastomers or foams, or for binding of fibers and/or particles.

## Revendications

1. Composé présentant deux motifs exovinylène-cyclocarbonate ou plus, les motifs exovinylène-cyclocarbonate étant liés les uns aux autres par l'intermédiaire d'au moins un groupe de liaison organique, exempt de siloxane, le groupe de liaison n'étant pas lié directement aux doubles liaisons exovinylène et les groupes de liaison formés par la polymérisation de monomères de (méth)acryle étant exclus, **caractérisé en ce que** le groupe de liaison présente au moins un groupe acétal et où il s'agit de composés de formule (Vb) 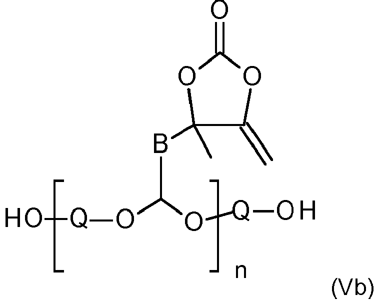 ou **en ce qu'**il s'agit de composés de formule (Vc) 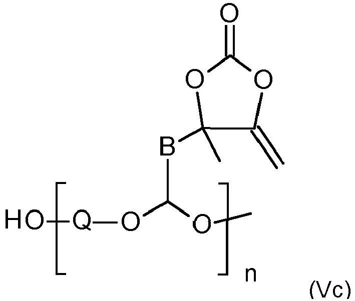 ou **en ce qu'**il s'agit de composés de formule (IVb) 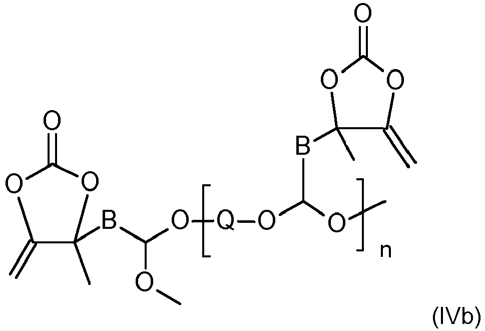 ou **en ce qu'**il s'agit de composés de formule (VI) 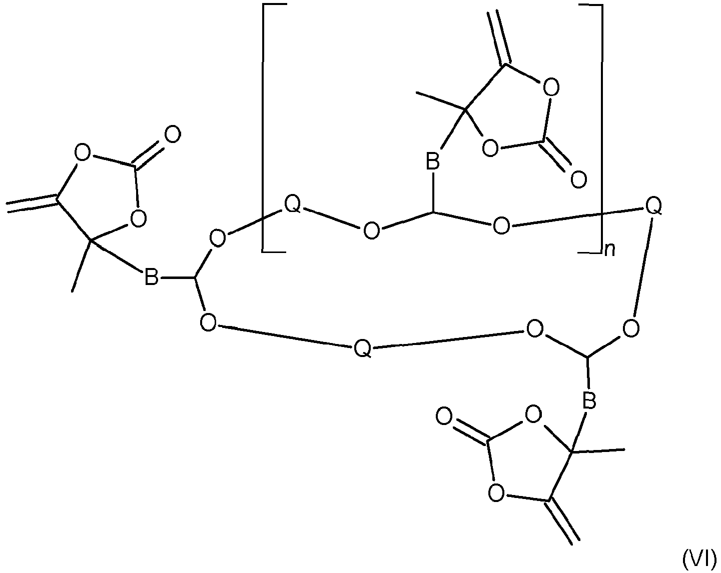 B signifiant un groupe alkylène comprenant de préférence 1 à 6 atomes de carbone ;
Q signifiant un groupe dérivé d'un polyol, un polyol étant un alcool présentant au moins deux groupes hydroxy ;
n dans les formules (Vb) et (Vc) étant un nombre supérieur ou égal à 2, de préférence de 2 à 10,
n dans la formule (IVb) étant un nombre supérieur ou égal à 1, de préférence de 1 à 10 et
n dans la formule (VI) étant un nombre supérieur ou égal à 0, de préférence de 0 à 10.

2. Composé selon la revendication 1, **caractérisé en ce qu'**il s'agit de composés de formule (VIa) 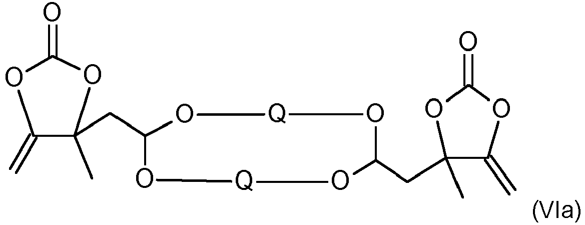 ou **en ce qu'**il s'agit de composés de formule (IVc) 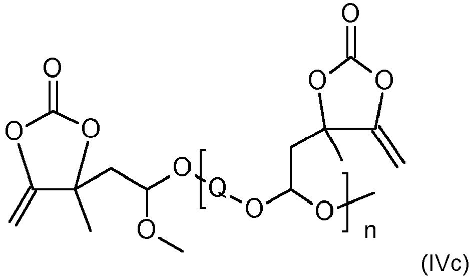 ou **en ce qu'**il s'agit de composés de formule (Vd) 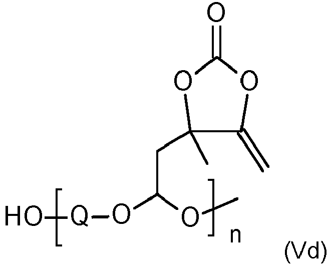 ou **en ce qu'**il s'agit de composés de formule (Ve) 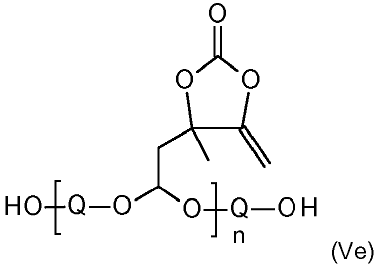
-O-Q-O- dans les formules VIa, IVc, Vd et Ve représentant à chaque fois le radical d'un alcool divalent, de préférence choisi parmi l'éthylèneglycol, le 1,3-propanediol, le 1,4-butanediol, le diéthylèneglycol, le triéthylèneglycol, le néopentylglycol, le 1,5-pentanediol, le 1,6-hexanediol, le 1,8-octanediol, le 3-méthyl-1,5-pentanediol, le 1,4-bis-hydroxyméthylcyclohexane, le 1,6-bis-hydroxyméthylcyclohexane, le glycérol, le diglycérol, le polyéthylèneglycol, le polypropylèneglycol, le poly-THF, le pentaérythritol, le dipentaérythritol, les alcools de sucre tels que le sorbitol et le mannitol,
n dans la formule (IVc) représentant 1, 2 ou 3 et
n dans les formules (Vd) et (Ve) représentant un nombre supérieur ou égal à 2, de préférence 2 ou 3.

3. Procédé pour la préparation d'un composé selon l'une quelconque des revendications 1 à 2, un composé, qui présente un motif exovinylène-cyclocarbonate et au moins un groupe acétal, étant transformé avec au moins un composé choisi dans le groupe constitué par les diols et les polyols.

4. Procédé selon la revendication précédente, **caractérisé en ce que** la transformation est réalisée sous catalyse avec des acides de Lewis ou des acides protoniques.

5. Utilisation d'une composition à deux composants comme liant pour des vernis, des peintures, des encres, des matériaux de construction, des élastomères, des mousses, des fibres ou des particules, un premier composant contenant au moins un composé selon l'une quelconque des revendications 1 à 2 et un deuxième composant contenant au moins un durcisseur multifonctionnel, qui présente au moins deux groupes fonctionnels choisis dans le groupe constitué par les groupes amino primaire, les groupes amino secondaire, les groupes hydroxy, les groupes phosphine, les groupes phosphonate et les groupes mercaptan.

6. Utilisation selon la revendication précédente, **caractérisée en ce que** la composition à deux composants contient au moins un catalyseur pour la catalyse de la réaction des groupes exovinylène-cyclocarbonate avec les groupes fonctionnels du durcisseur.

7. Utilisation d'un composé selon l'une quelconque des revendications 1 à 2 comme constituant d'agents de revêtement, de vernis, de peintures, d'encres, de matériaux de construction, d'élastomères, de mousses ou pour la liaison de fibres et/ou de particules.
